# EUROPEAN PATENT APPLICATION

(11) **EP 4 678 656 A1**
(43) Date of publication of application: **14.01.2026**
(21) Application number: 24187641.6
(22) Date of filing: 10.07.2024
(51) Int. Cl.: C07K 14/50, C07K 14/705, C12N 5/077, C12N 5/071

(54) **METHODS FOR PRODUCING ATRIOVENTRICULAR CANAL (AVC)-LIKE CARDIOMYOCYTES**

(71) Applicant: Stichting Amsterdam UMC, 1081 HV Amsterdam (NL)
(72) Inventor: The designation of the inventor has not yet been filed
(74) Representative: de Pauw, Elmar Sebastian David

(57) **Abstract**

The present disclosure provides a method of generating AVC-like cell(s) or AVC-like cardiomyocyte(s) from cardiogenic mesoderm cells comprising incubating the cardiogenic mesoderm cells in an AVC cardiomyocyte induction medium comprising WNT2 and retinoic acid (RA) to obtain the AVC-like cell(s). Preferably, the AVC-like cell(s) express *TBX3* and at least one of *BMP2, MSX2* and *TBX2.*

## Description

### TECHNICAL FIELD

The present disclosure provides compositions and methods for generating cells with Atrioventricular Canal (AVC) characteristics from pluripotent stem cells or cardiogenic mesoderm cells.

### BACKGROUND

No robust methods are currently available for the generation of AVC cardiomyocytes from hPSCs. In the recent years, protocols have been described for the generation of atrial (Devalla et al, 2015; Li et al, 2022) and sinoatrial nodal cardiomyocytes (Protze et al, 2017; Wiesinger et al, 2022) and ventricular cardiomyocytes (Funakoshi et al, Nat Commun. 2021 May 26;12(1):3155. doi: 10.1038/s41467-021-23329-z; Dark et al, Cell Rep Methods. 2023 Apr 24;3(4):100456. doi: 10.1016/j.crmeth.2023.100456.) from human induced pluripotent stem cells but a reliable method for the generation of AVC-like cells is lacking. A recent study reported the generation of AVC-like cells by modulating the generation of mesoderm, which subsequently yields AVC-like cells upon exposure to BMP ligand, BMP4 (Schmidt et al.). Although this approach is promising, AVC-like cells generated in this study do not display a key characteristic, i.e., slower impulse propagation compared with atrial and ventricular-like cells. This approach is also met with challenges such as complexity and reproducibility.

The present disclosure thus sets out to address the lacunae in the art and provide a method for the generation of AVC-like cardiomyocytes from pluripotent stem cells or mesoderm cells.

### BRIEF DESCRIPTION OF THE FIGURES

In order that the disclosure may be readily understood and put into practical effect, reference will now be made to exemplary embodiments as illustrated with reference to the accompanying figures. The figures together with detailed description below, are incorporated in and form part of the specification, and serve to further illustrate the embodiments and explain various principles and advantages, where:

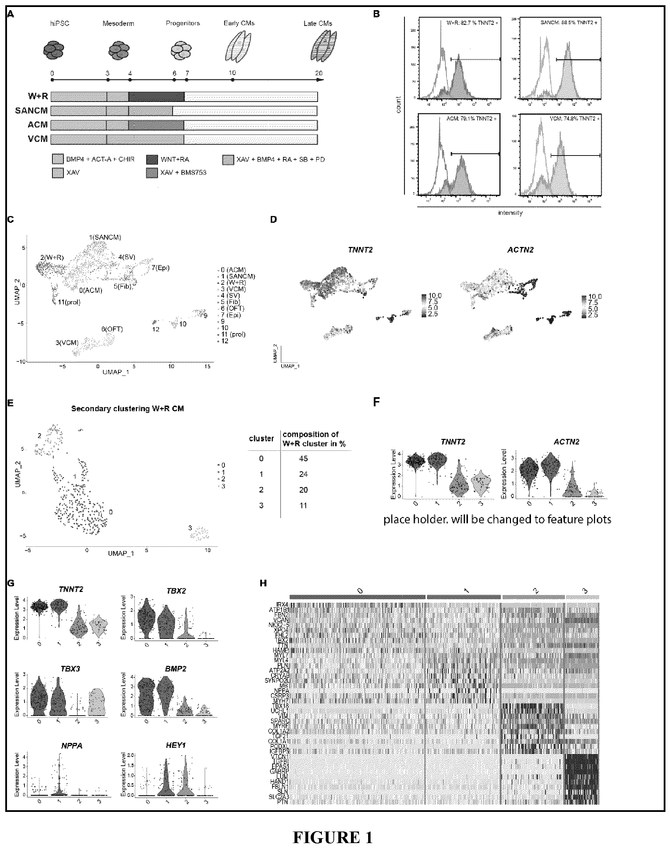

**Figure 1** depicts gene expression profiling of cardiomyocytes obtained from W+R treatment. (A) Schematic of the directed differentiation protocols to steer human induced pluripotent stem cells (hiPSCs) to atrioventricular (AV) canal cardiomyocytes by treatment with WNT2 and Retinoic Acid, labeled W+R. Directed differentiation approaches to steer other cardiomyocyte subtypes, i.e., sinoatrial nodal (SANCM), Atrial (ACM) and Ventricular cardiomyocytes (VCM) are also illustrated. (B) Representative histograms showing proportion of TNNT2+ cells at day 19 in the different groups. (C) UMAP representation of single cell transcriptomes of W+R, SANCM, ACM, and VCM differentiation cultures (D) Feature plots demonstrating expression of *TNNT2* and *ACTN2* in cell populations obtained from W+R, SANCM, ACM, and VCM cultures (E) UMAP representation of single cell transcriptomes of W+R cultures (Left). Quantification of % of cells found in each cluster (Right) (F) Feature plots demonstrating expression of *TNNT2* and *ACTN2* in clusters obtained from W+R treatment. (G) Violin plots showing expression of *TNNT2, TBX2, TBX3, BMP2, NPPA* and *HEY1* in clusters obtained from W+R treatment. (H) Heatmap showing top 10 genes differentially expressed in clusters obtained from W+R treatment. Epi, (Pro)epicardial cells; Fib, Fibroblasts; OFT, Outflow tract cardiomyocytes; Prol, Proliferating cardiomyocytes; SV, Sinus venosus cardiomyocytes.

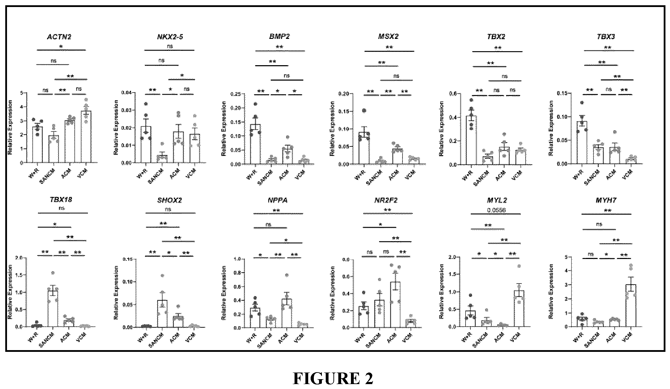

**Figure 2** depicts Gene expression in hiPSC-derived cardiomyocyte subtypes. qRT-PCR showing expression of cardiac (ACTN2, NKX2-5), AV canal (BMP2, MSX2, TBX2, TBX3), sinoatrial nodal (TBX18, SHOX2), atrial (NPPA, NR2F2), and ventricular (MYL2, MYH7) enriched genes in cells taken on day 19 of differentiation. n = 5 independent differentiations. Error bars, s.e.m. Statistical Test, Kruskal Wallis, Mann-Whitney test. *P<0.05, **P<0.01. W+R, WNT+RA treated cardiomyocytes; SANCM, sinoatrial nodal cardiomyocytes; ACM, Atrial cardiomyocytes; VCM, Ventricular cardiomyocytes.

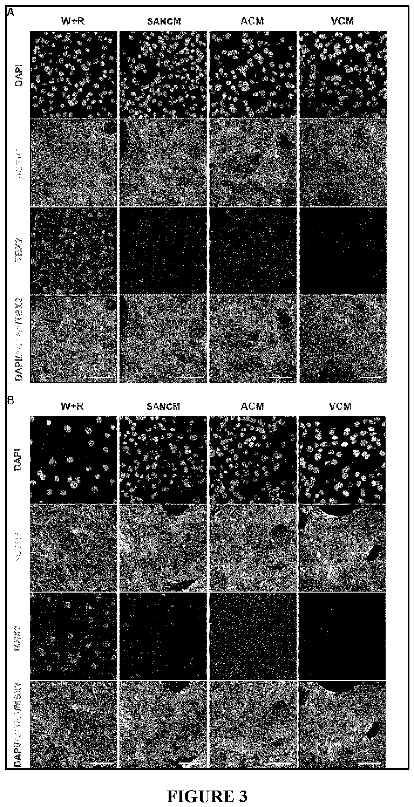

**Figure 3** depicts Expression of TBX2 and MSX2 in hiPSC-derived cardiomyocyte subtypes. (A-B) Immunostaining for (A) ACTN2 and TBX2 and (B) ACTN2 and MSX2 in W+R, SANCM, ACM and VCM cardiomyocyte cultures. Scale bar 100 µm.

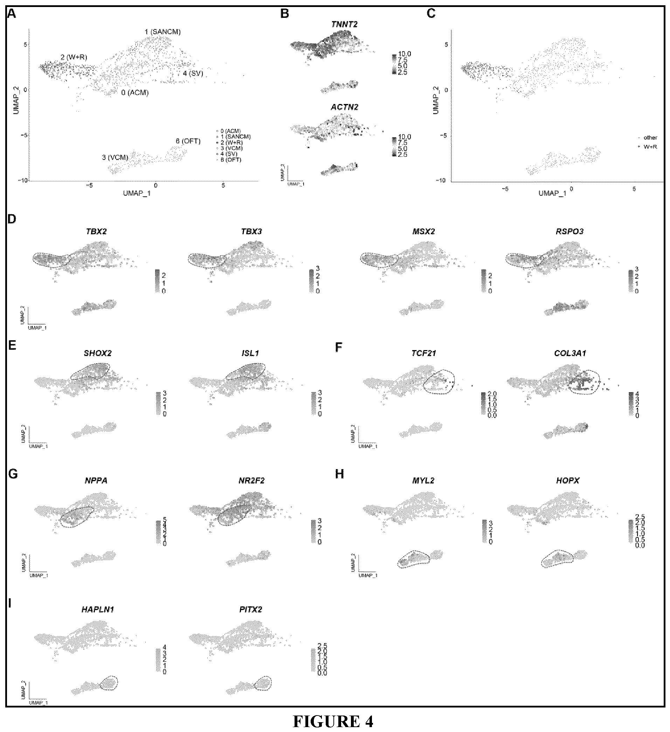

**Figure 4** depicts single cell RNA sequencing of hiPSC-derived cardiomyocyte subtypes. (A) UMAP projection of single cell transcriptomes of cardiomyocyte only groups from subtype populations. (B) Feature plots showing expression of TNNT2 and ACTN2 in all cardiomyocyte clusters. (C) Annotation of cells obtained from WNT+ RA (W+R) treated cultures on the UMAP containing all hiPSC-derived cardiomyocyte subtypes (D - I) Feature plots showing expression of genes associated with (D) atrioventricular canal cardiomyocytes (E) Sinoatrial nodal cardiomyocytes, SANCM (F) Sinus venosus cardiomyocytes, SV (G) atrial cardiomyocytes, ACM (H) ventricular cardiomyocytes, VCM and (I) outflow tract cardiomyocytes, OFT genes that facilitated assignment of identities to the different clusters.

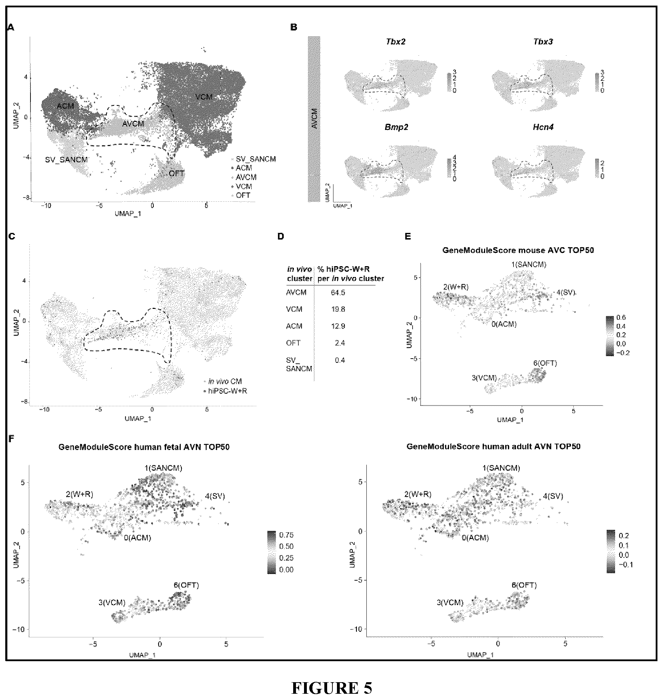

**Figure** 5 depicts comparative transcriptomic analysis of W+R cardiomyocytes with murine and human AV nodal cardiomyocytes. (A) UMAP representation of cardiomyocyte clusters obtained from single cell transcriptomes of murine heart using datasets reported in de Soysa et al, 2019 and Hill et al, 2019. (B) Feature plots showing expression of atrioventricular (AV) canal associated genes Tbx2, Tbx3, Bmp2, and Hcn4. (C) Annotation of hiPSC-derived W+R cardiomyocytes (Red dots) on the in vivo dataset showing distribution across clusters. (D) Quantification of % of W+R cells found in clusters containing in vivo cardiomyocyte subtypes. (E) Gene module scoring (GMS) of hiPSC-derived cardiomyocyte subtype datasets utilized this study (hiPSC-CM) using Top 50 genes expressed in mouse AV canal. (F) GMS of hiPSC-CM using Top 50 genes of human fetal (Left) and human adult (Right) AV nodal cells. ACM, Atrial cardiomyocytes; AVCM, AV canal cardiomyocytes; OFT, Outflow tract cardiomyocytes; SANCM, Sinoatrial nodal cardiomyocytes; SV, Sinus venosus cardiomyocytes; VCM, Ventricular cardiomyocytes; W+R, WNT+RA treated cardiomyocytes.

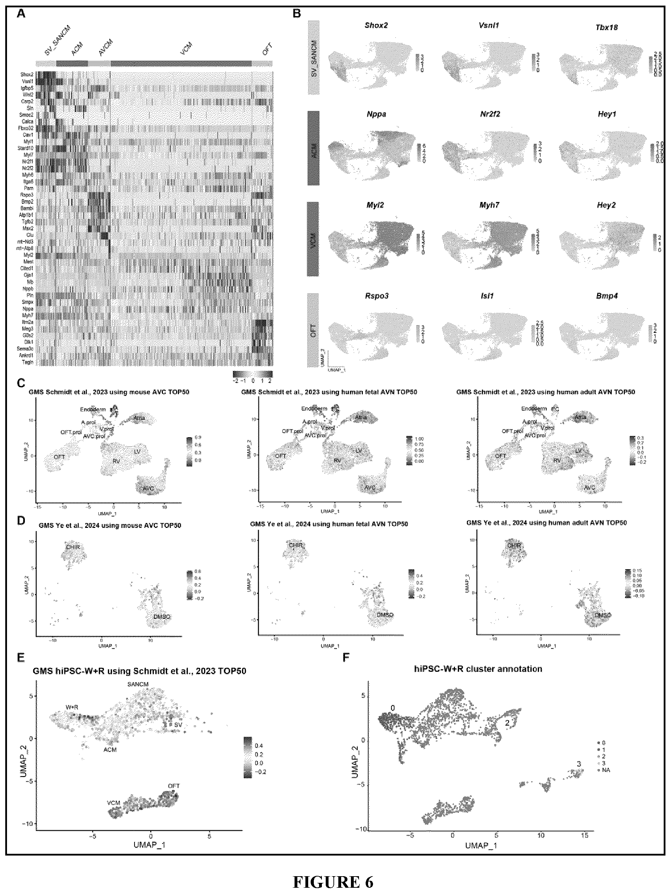

**Figure 6** depicts Comparison of single cell profiles of W+R cells to available in vivo and in vitro datasets. (A) Heatmap showing top 10 genes expressed in clusters identified in murine heart datasets. (B) Feature plots showing expression of genes associated with sinus venosus and sinoatrial nodal cardiomyocytes (SV_SANCM), atrial cardiomyocytes (ACM), ventricular cardiomyocytes (VCM) and outflow tract cardiomyocytes (OFT) that facilitated assignment of identities to the different clusters. (C) Gene module scoring of hiPSC-derived cell clusters including AVCM reported in Schmidt et al using Top 50 AV canal/node genes from murine (Left), human fetal (Center) and human adult heart (Right). (D) Gene module scoring of hiPSC-derived cell clusters including AVCM reported in Ye et al. using Top 50 AV canal/node genes from murine (Left), human fetal (Center) and human adult heart (Right) (E) Gene module scoring of hiPSC-derived cardiomyocyte subtypes including AVCM reported in this study using Top 50 genes expressed in hiPSC-derived AVCM of Schmidt et al. (F) Annotation of cardiomyocyte Clusters 0 and 1 (from Figure 1E) in W+R cultures on the UMAP of hiPSC-derived cardiomyocyte subtypes including AVCM reported in this study.

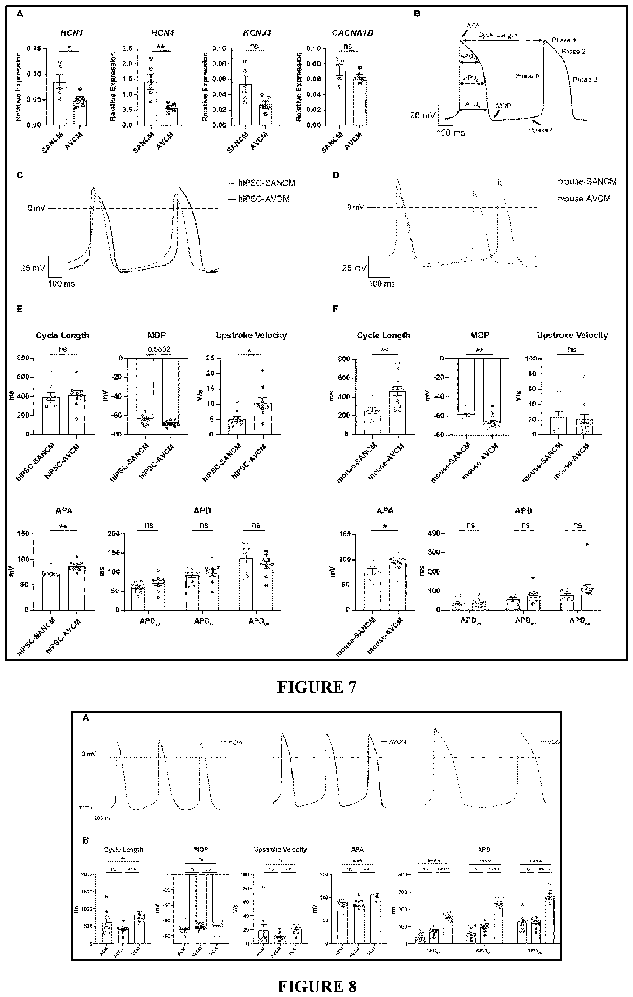

**Figure 7** depicts electrophysiological characterization of hiPSC-derived AVCM. Expression of HCN1, HCN4, KCNJ3 and CACNA1D in hiPSC-derived SANCM and AVCM. n = 5 independent differentiations (B) Illustration of analyzed action potential parameters using single cell patch-camp methodology (C - D) Representative action potential traces of (C) hiPSC-derived SANCM, AVCM, and (D) isolated E17.5 murine SANCM and AVCM. (E - F) Quantified action potential parameters in (E) hiPSC-derived SANCM, AVCM, and (F) isolated E17.5 murine SANCM and AVCM. n = 9 hiPSC-cell types from three independent differentiations; n = 9 murine SANCM, n = 14 murine AVCM. Error bars, s.e.m. Statistical Test, Kruskal Wallis, Mann-Whitney test: *P<0.05, **P<0.01, ***P<0.001, ****P<0.0001. SANCM, Sinoatrial nodal cardiomyocytes; AVCM, Atrioventricular canal cardiomyocytes; MDP = Maximal diastolic depolarization; APA = Action potential amplitude; APD20, APD50, APD90 = Action potential duration (APD) at 20%, 50%, 90% repolarization.
**Figure 8** depicts electrophysiological characterization of hiPSC-ACM, AVCM and VCM. (A) Representative action potential traces of cardiomyocyte subtypes (B) Quantification of action potential parameters. n = 9 from three independent differentiations. Error bars s.e.m. Statistical Test. Kruskal Wallis, Mann Whitney. *P<0.05, **P<0.01, ***P<0.001, ****P<0.0001. ACM, Atrial cardiomyocytes; AVCM, Atrioventricular cardiomyocytes; VCM Ventricular cardiomyocytes. MDP, maximal diastolic potential; APA, action potential amplitude; APD, action potential duration (APD) at 20 (APD20), 50 (APD50), 90 (APD90) % of repolarization.

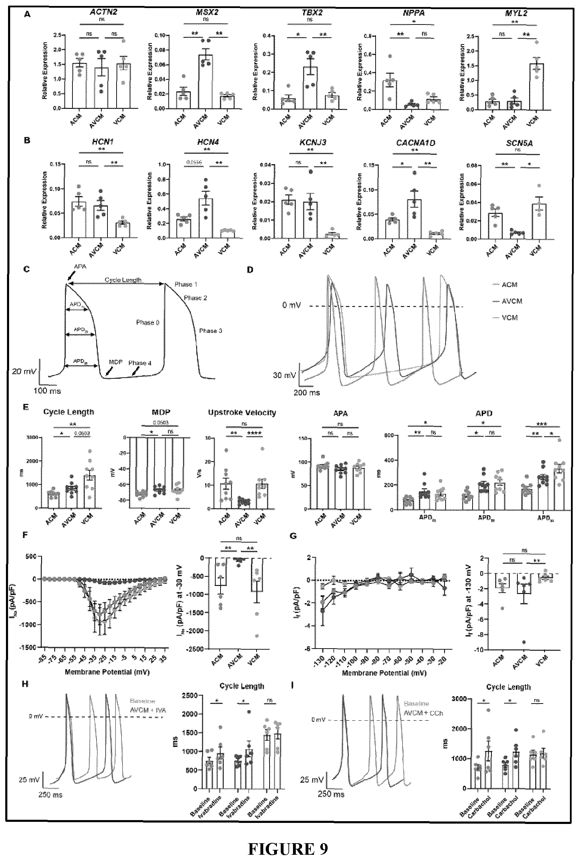

**Figure 9** depicts generation of AVCM from an independent hiPSC line. qRT-PCR showing expression of (A) cardiac (ACTN2), subtype-specific and (B) ion channel genes in ACM, AVCM, and VCM generated from hiPSC line LUMCiCTRL0047#1A. n = 5 independent differentiations (C) Illustration of analyzed action potential parameters by single cell patch-clamp. (D) Representative traces of ACM, AVCM, and VCM spontaneous action potentials. (E) Quantification of action potential parameters in cardiomyocyte subtypes. n = 9 from three independent differentiations. (F) Current voltage (I-V) relationships of the sodium current (INa)(Left); INa density recorded at -30mV (voltage at which INa is at a maximum) in cardiomyocyte subtypes (Right). n = 6 from three independent differentiations. (G) I-V relationships of the hyperpolarization-activated funny current (If) (Left); If density recorded at -130 mV, (voltage at which If is at a maximum) in cardiomyocyte subtypes (Right). n = 6 from three independent differentiations. (H) Representative action potentials of AVCM (Left) and cycle length quantification (Right) of response to 3 µM ivabradine (IVA). n = 6 from three independent differentiations. (I) Representative action potentials of AVCM (Left) and cycle length quantification (Right) of response to 10 µM carbachol (CCh). n = 6 from three independent differentiations. Error bars s.e.m. Statistical Test Kruskal Wallis, Mann-Whitney for Fig 9A, 9B, 9E, 9F, 9G. Wilcoxon for Fig 9F, 9H, 9H, 9I. *P<0.05, **P<0.01, ***P<0.001. ACM, Atrial cardiomyocytes; AVCM, Atrioventricular canal cardiomyocytes; VCM, Ventricular cardiomyocytes; MDP = Membrane diastolic depolarization; APA= Action potential amplitude; APD20, APD50, and APD90 = Action potential duration (APD) at 20%, 50%, and 90% repolarization.

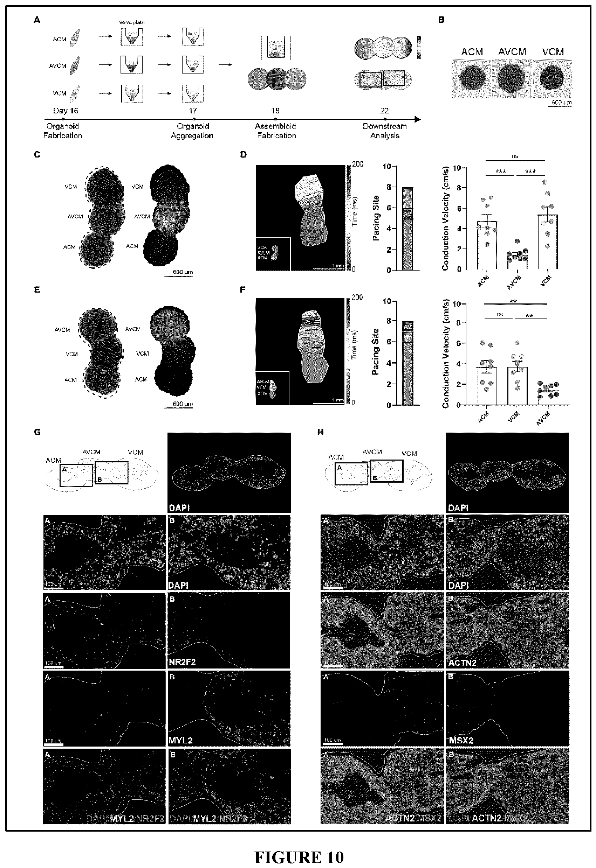

**Figure 10** depicts generation of cardiac assembloids to simulate AV conduction axis. (A) Graphical illustration of the approach to generate assembloids from subtype-specific spheroids. (B) Representative brightfield images of subtype-specific spheroids on day 18, scale bar 600 µm. (C) Brightfield and fluorescent images of a representative example of an assembloid fabricated with AVCM (labeled with GFP) sandwiched between ACM and VCM. scale bar 600 µm. (D) Activation map of assembloid containing AVCM sandwiched between ACM and VCM (Left), scale bar 1 mm; Stacked bar graph demonstrating pacing site (Center); Conduction velocity measured in subtype segments of the assembloids (Right). n = 8 from three independent differentiations. (E) Brightfield and fluorescent images of a typical example of an assembloid fabricated with VCM sandwiched between ACM and AVCM (labeled with GFP). Scale bar 600 µm. (F) Activation map of assembloid containing VCM sandwiched between ACM and AVCM (Left), scale bar 1 mm; Stacked bar graph demonstrating pacing site (Center); Conduction velocity measured in subtype segments of the assembloids (Right). n = 8 from three independent differentiations. (G-H) Immunostaining of assembloids for (G) DAPI, NR2F2, and MLY2 (H) DAPI, ACTN2, and MSX2. Scale bar 100 µm. *P<0.05, **P<0.01, ***P<0.001. ACM, Atrial cardiomyocytes; AVCM, Atrioventricular cardiomyocytes; VCM, Ventricular cardiomyocytes.

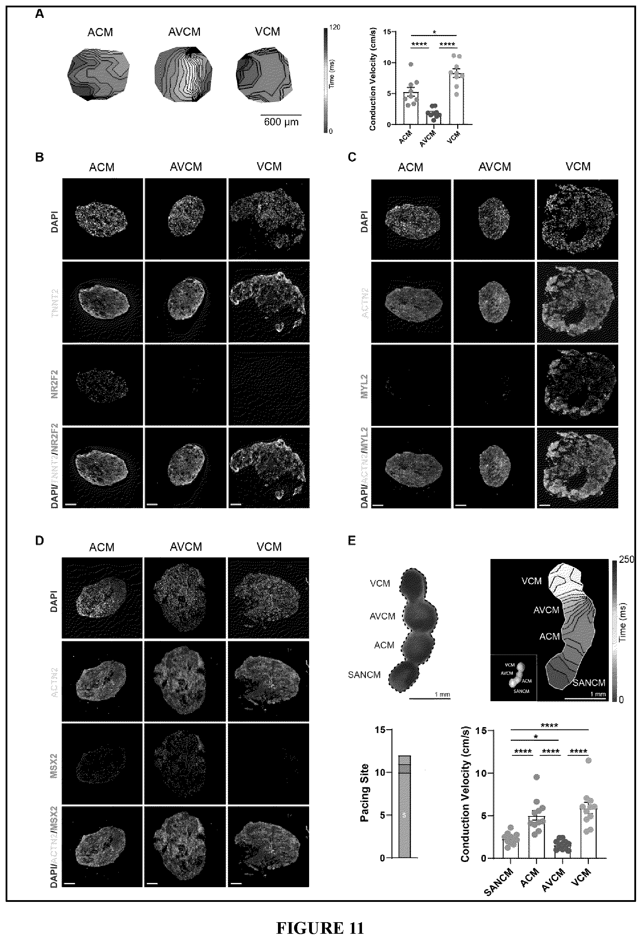

**Figure 11** depicts characterization of subtype organoids and assembloids. (A) Representative activation maps (Left) and average conduction velocities (Right) of spheroids made from individual cardiomyocyte subtypes. n = 9 from two independent differentiations. (B-D) Immunofluorescence staining of subtype spheroids for (B) DAPI, TNNT2 and NR2F2 (C) DAPI, ACTN2 and MYL2 (D) DAPI, ACTN2 and MSX2. Scale bar 200 µM. (E) Representative brightfield image (Top Left) and activation map (Top Right) of a quad assembloid. Stacked bar graph quantifying location of pacing site in quad assembloids (Lower Left). Conduction velocities measured in subtype segments of the quad assembloids (Lower Right). Error bars, s.e.m. Statistical Test, Kruskal Wallis, Mann-Whitney test: *P<0.05, **P<0.01, ***P<0.001, ****P<0.0001. SANCM, Sinoatrial nodal cardiomyocytes; ACM, Atrial cardiomyocytes, AVCM, Atrioventricular cardiomyocytes; VCM Ventricular cardiomyocytes.

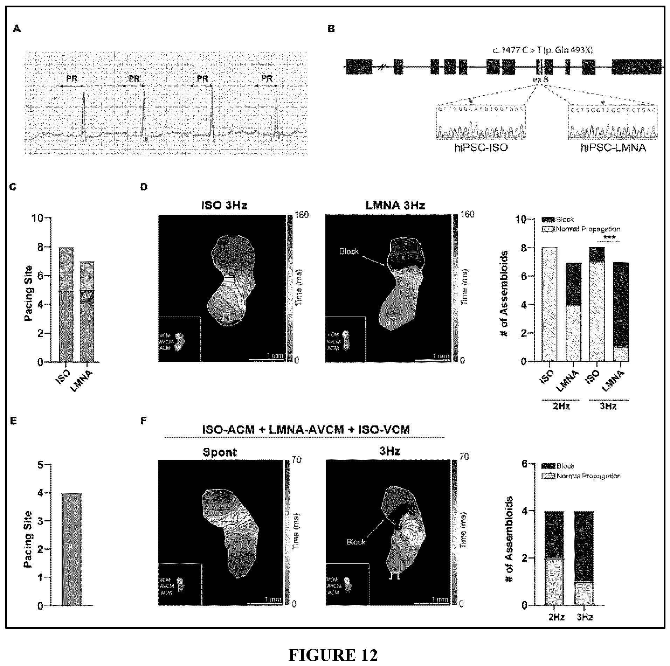

**Figure 12** depicts cardiac assembloids model conduction dysfunction in LMNAc.1477C>T hiPSCs. (A) ECG trace of a patient carrying LMNAc.1477C>T variant. Arrows denote PR interval. (B) DNA sequence demonstrating the presence of C>T substitution at position 1477 (red arrow) in patient-specific hiPSCs (right panel) and correction of this variant by prime editing resulting in an isogenic control line (left panel). (C) Stacked bar graph demonstrating location of pacing site in ACM-AVCM-VCM assembloids generated from LMNAc.1477C>T iPSCs and mutation corrected isogenic iPSCs, n = 7 - 8 from three independent differentiations. (D) Stacked bar graph quantifying impulse block observed in isogenic and LMNAc.1477C>T assembloids paced at 2 Hz and 3 Hz, n = 7 - 8 from three independent differentiations. (E) Representative activation maps of isogenic and LMNAc.1477C>T assembloids paced at 3 Hz. (F) Bar graph demonstrating location of pacing site in ACM-AVCM-VCM assembloids. ACM and VCM in this setting were generated from isogenic control cells while AVCM were generated from LMNAc.1477C>T cells. n = 4 from three independent differentiations. (G) Stacked bar graph quantifying impulse block observed in mixed assembloids paced at 2 Hz and 3 Hz, n = 4 from three independent differentiations. (H) Representative activation maps of mixed assembloids, spontaneous (Left) and paced at 3 Hz (Right).

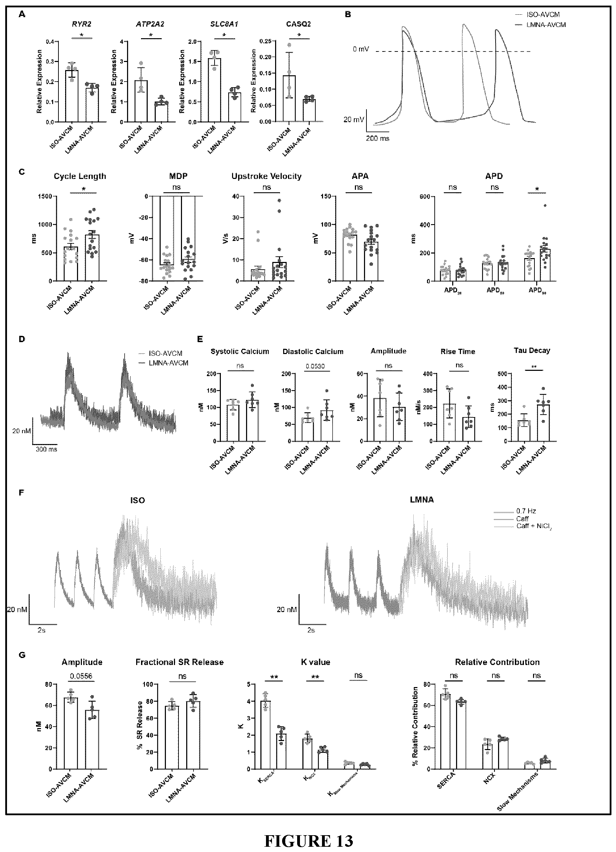

**Figure 13** depicts LMNAc.1477C>T exhibit defects in intracellular calcium handling. (A) qRT-PCR showing expression of RYR2, ATP2A2 (SERCA2A), SLC8A1 (NCX1), and CASQ2 in AVCM from isogenic and LMNAc.1477C>T AVCM cells. n = 4 independent differentiations. (B) Representative action potential traces of isogenic and LMNAc.1477C>T AVCM. (C) Quantification of action potential parameters, n = 17 from three independent differentiations. (D) Representative traces of intracellular calcium ([Ca2+]i) transients in Indo-1 AM-loaded AVCM paced at 0.7Hz. (E) Quantified calcium transient parameters in AVCM, n = 7 from three independent differentiations. (F) Representative traces of [Ca2+]i transients in Indo-1 AM-loaded AVCM paced at 0.7 Hz, in the presence of caffeine (Caff) or caffeine + nickel chloride (Caff+NiCl2). (G) Amplitude of [Ca2+]i in presence of caffeine and NiCl2 and fractional sarcoplasmic reticulum (SR) release, Rate constants (K) of SERCA-, NCX-, and slow-mechanism-based [Ca2+]i decay, Relative contribution of SERCA, NCX and slow mechanisms to [Ca2+]i extrusion. n = 5 from three independent differentiations. Error bars, s.e.m. Statistical Test, Kruskal Wallis, Mann-Whitney test: *P<0.05, **P<0.01, ***P<0.001, ****P<0.0001.

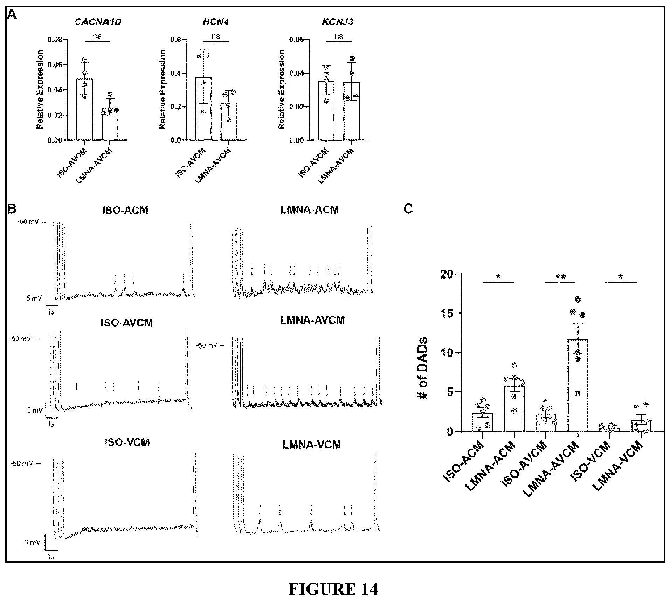

**Figure 14** depicts characterization of isogenic and LMNAc.1477C>T cardiomyocyte subtypes. (A) Expression of CACNA1D, HCN4, and KCNJ3 in isogenic and LMNAc.1477C>T AVCM. n = 4 independent differentiations. (B) Typical examples demonstrating delayed afterdepolarization (DAD) inducibility in ACM, AVCM and VCM from isogenic and LMNAc.1477C>T hiPSCs. Arrows point to DADs. (C) Quantification of DADs in ACM, AVCM and VCM from isogenic control and LMNAc.1477C>T ACM, AVCM, and VCM. n = 6 from three independent differentiations. Error bars s.e.m. Statistical Test. Kruskal Wallis, Mann Whitney. *P<0.05, **P<0.01. ACM, Atrial cardiomyocytes; AVCM, Atrioventricular cardiomyocytes; VCM Ventricular cardiomyocytes.

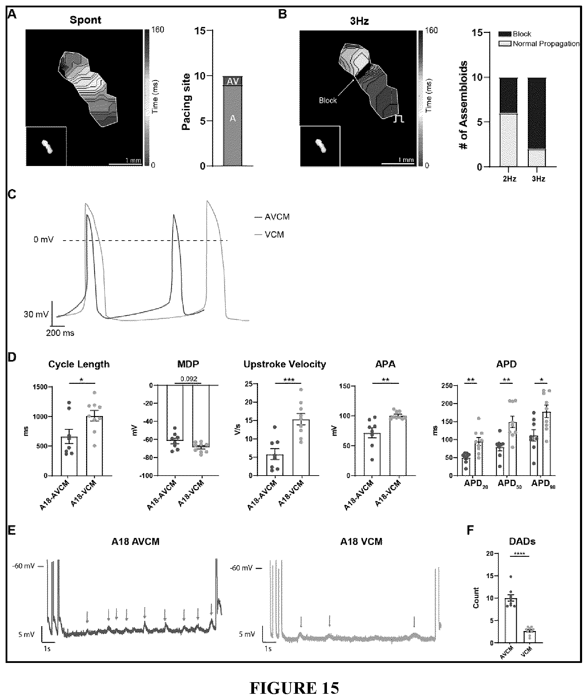

**Figure 15** depicts electrophysiological characterization of an independent clone (A18) harboring LMNAc.1477C>T. (A) Representative activation map (Left) and stacked bar graph quantifying pacing site (Right) in assembloids generated from A18 LMNAc.1477C>T clone. (B) Representative activation map of an assembloid paced at 3 Hz (Left) and stacked bar graph (Right) quantifying impulse block in assembloids paced at 2 Hz and 3 Hz, n = 10 from three independent differentiations. (C) Representative action potential traces and (D) Quantification of action potential parameters of AVCM and VCM from A18 LMNAc.1477C>T clone. n = 8 three independent AVCM differentiations. n = 9 three independent VCM differentiation (E) Typical example demonstrating inducibility of delayed afterdepolarizations (DAD) in AVCM and VCM. Arrows point to DADs. (F) Quantification of DADs in AVCM and VCM. n = 9 three independent differentiations. Error bars s.e.m. Statistical Test. Kruskal Wallis, Mann Whitney. *P<0.05, **P<0.01. AVCM, Atrioventricular cardiomyocytes; VCM Ventricular cardiomyocytes.

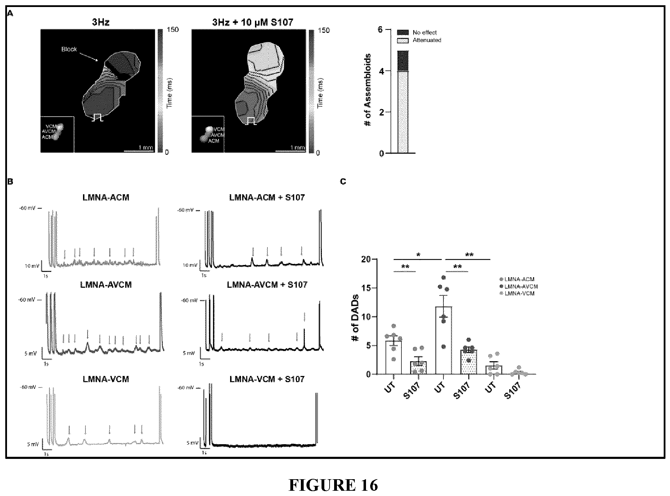

**Figure 16** depicts rycal stabilizer S107 mitigates conduction block in LMNAc.1477C>T AVCM. (A) Representative activation maps of LMNA assembloids (Left) before and after treatment with 10 µM S107. Quantification of impulse block in assembloids. n = 5 from three independent differentiations. (B) Representative examples and (C) quantification of delayedafterdepolarization (DAD) inducibility before and after treatment with S107 in atrial cardiomyocytes (ACM), atrioventricular cardiomyocytes (AVCM) and ventricular cardiomyocytes (VCM). n = 6 from three independent differentiations. Error bars s.e.m. Statistical Test. Kruskal Wallis, Mann Whitney. *P<0.05, **P<0.01.

### SUMMARY OF THE INVENTION

Addressing the aforesaid need in the art, the present disclosure provides a method of generating AVC-like cell(s) from cardiogenic mesoderm cells comprising:
incubating the cardiogenic mesoderm cells in an AVC cardiomyocyte induction medium comprising WNT2 and retinoic acid (RA) to obtain AVC-like cell(s), wherein the AVC-like cell(s) express *TBX3* and at least one of *BMP2, MSX2, TBX2.*

Preferably, said cardiogenic mesoderm cells express MESP1 and/or MESP2 and optionally, EOMES.

In some embodiments, said cardiogenic mesoderm cells may, optionally, further express EOMES.

Thus, in some embodiments, said cardiogenic mesoderm cells express MESP1 and/or MESP2 and optionally, EOMES.

In some embodiments, the AVC cardiomyocyte induction medium comprises about 1 ng/mL to about 20 ng/mL WNT2 and about 50 nM to about 1000 nM RA.

In some embodiments, the AVC cardiomyocyte induction medium may further comprise a BMP ligand, wherein non-limiting examples of the BMP ligand include *BMP4* and *EMP2.*

In some embodiments, the cardiogenic mesoderm cells are incubated in the AVC cardiomyocyte induction medium comprising WNT2, retinoic acid (RA) and optionally, a BMP ligand such as but not limited to *BMP4* and/or *BMP2* for about 24 hours to about 96 hours.

In some embodiments, the MESP1/2 mesoderm cells are prepared by incubating a pluripotent stem cell (PSC) in an early mesoderm induction medium comprising one or more of a BMP ligand, a nodal signalling protein (TGFb superfamily protein) and activators of WNT signalling to generate MESP1/2 mesoderm cells expressing MESP1 and/or MESP2 and optionally, EOMES.

The early mesoderm induction medium may be any medium that is routinely used for culturing animal cells can be used. In some embodiments, the early mesoderm induction medium is selected from a group comprising APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media. In some embodiments, non-growth factor additives, such as antibiotics, B-27 supplement (with or without insulin), amino acids, salts, ascorbic acid and thioglycerol may be added to the said early mesoderm induction medium.

Accordingly, in some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein to generate MESP1/2 mesoderm cells; and
incubating the MESP1/2 mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

Further provided in the present disclosure are AVC-like cell(s) obtainable by the method(s) as described above.

The present disclosure also provides AVC-like cell(s) or a population of AVC-like cell(s) expressing *TBX3* and at least one of *BMP2, MSX2, TBX2.*

The present disclosure further provides an engineered tissue comprising the AVC-like cell(s) as described above.

In some embodiments, the engineered tissue further comprises Sinoatrial nodal cardiomyocytes (SANCM), Atrial cardiomyocytes (ACM) and Ventricular cardiomyocytes (VCM).

In some embodiments, the said engineered tissue is an organoid containing the AVC-like cell(s) (AVCM), Atrial cardiomyocytes (ACM) and/or Ventricular cardiomyocytes (VCM).

In some embodiments, the engineered tissue is an assembloid containing SANCM, AVCM, ACM and/or VCM organoids.

In terms of intended application of the AVC-like cell(s), in some embodiments, the present disclosure provides a method of identifying a candidate drug comprising:
a. generating AVC-like cell(s) according to the method(s) as described above;
b. contacting the AVC-like cell(s) with a drug candidate;
c. measuring parameter(s) selected from a group comprising beat rate, action potential characteristics and/or ion currents of the cardiomyocytes or AVC-like cell(s) contacted with the drug candidate;
d. comparing the said parameter(s) to a control AVC-like cell(s) not treated with the drug candidate; and
e. selecting the drug candidate which modulates the said parameter(s) compared to the control cell as the candidate drug.

Further envisaged herein are AVC-like cell(s) as obtained by the method(s) as described above, for use as a medicament or in disease modelling.

The present disclosure also covers use of the AVC-like cell(s) obtained by the method(s) described herein, in a drug discovery method.

### DETAILED DESCRIPTION

The present disclosure provides a developmentally guided differentiation approach for the generation of cardiomyocytes, particularly atrioventricular canal (AVC)-like cardiomyocytes from pluripotent stem cells (PSCs).

However, before describing the subject matter of the present disclosure in further detail, the below paragraphs set out definitions for some terms used throughout the present disclosure for the purposes of clarity and convenience. Unless defined otherwise, all technical and scientific terms used herein have the same meaning as commonly understood by one of ordinary skill in the art to which this present disclosure belongs. Although any methods and materials similar or equivalent to those described herein can be used in the practice or testing of the present invention, the preferred methods and materials are now described.

### Definitions

The terms "express" and "expression level" as used herein may refer to expression of genes or proteins.

The term "cardiomyocyte" as used herein is a cardiac lineage cell that expresses cTNT and *ACTN2.*

The term "atrioventricular canal -like cardiomyocyte" or "AVC-like cardiomyocyte" or "AVC-like cells" or AVCM as interchangeably used herein refers to cardiomyocytes which exhibit electrophysiological properties of AVC cardiomyocytes which are capable of displaying "funny current", I_{f} or response to ivabradine. The AVC-like cardiomyocytes express *TBX3* and at least one or two of markers *BMP2, MSX2, TBX2.* The AVC-like cardiomyocytes can initiate beating in certain conditions, and they display a "funny current", which is a mixed sodium-potassium current that activates upon hyperpolarization at voltages in the range of -130 mV to -20 mV). The funny current flows through an HCN-dependent channel. Notable electrophysiological properties of AVC-like cells in comparison with atrial or ventricular-like cardiomyocytes are a) shorter cycle length and b) slower upstroke velocity. Furthermore, similar to *in vivo* AVC cells, AVC-like cardiomyocytes *in vitro* show responses to ivabradine, which targets funny current, I_{f} or exhibition of HCN dependent funny current and response to carbachol, which activates inward rectifier potassium current, I_{KACH}.

The term "sinoatrial nodal cardiomyocyte" (SANCM) as used herein refers to a cardiomyocyte expressing *TBX18, SHOX2* and *ISL1.*

The term "atrial cardiomyocyte" (ACM) as used herein refers to a cardiomyocyte expressing *NKX2-5, NPPA* and *NR2F2.*

The term "ventricular cardiomyocyte" (VCM) as used herein refers to a cardiomyocyte expressing *NKX2-5, MYL2* and *MYH7.*

The terms "MESP1 mesoderm cells", "MESP2 mesoderm cells" or "MESP1/2 mesoderm cells" as used herein refers to cells which may be produced from pluripotent stem cells (PSC) and have the following characteristics:
- express MESP1 and/or MESP2, as detected by quantitative real-time PCR as described herein, and
- express NANOG at a lower level compared to PSC and preferably express OCT4 at a lower level than PSC after 3 days of culturing in a late mesoderm induction medium can differentiate into cardiomyocytes.

The term "WNT inhibitor" as used herein means any agent, including any compound and/or protein that inhibits WNT signalling, including but not limited to WNT antagonists that bind either to the WNT ligand itself, or to WNT receptors. Non-limiting examples of WNT inhibitors include Dickkopf (Dkk) proteins, WNT Inhibitory Factor-1 (WIF-1), and secreted Frizzled-Related Proteins (sFRPs), as well as WNT inverse agonists (e.g. an agent that binds to the same receptor as an agonist but induces a pharmacological response opposite to that of an agonist). Other such examples of WNT inhibitors include XAV939, IWP 2, an inhibitor of wnt processing, IWR2, WNT-C-59and iCRT14, which is a potent inhibitor of β-catenin-responsive transcription (CRT), as well as combinations thereof.

The term "activator of WNT signalling" as used herein means any agent, including any compound and/or protein that stimulates WNT signalling. Examples of such activators of WNT signalling include but are not limited to CHIR 99021, BIO, LY2090314, DCA.

The term "ROR1+ and ROR2+ mesoderm cell" as used herein refers to mesoderm cells having the following characteristics:
express ROR1 and ROR2 (i.e. are ROR1 and ROR2 positive), as detected by flow cytometry as described herein, and
can differentiate into cardiomyocytes.

The term "BMP ligand" as used herein means any molecule such as any BMP or growth and differentiation factor (GDF) that activates the type I and type II serine-threonine kinase receptors including *EMPRIA* (ALK3), *BMPR1B* (ALK6), including for example *BMP4* and *BMP2.*

The term *"BMP4"* (for example Gene ID: 652) as used herein refers to Bone Morphogenetic Protein 4, for example human *BMP4,* as well as active conjugates and fragments thereof, that can for preferably activate *BMP4* receptor signalling.

The term "retinoic acid" or "RA" includes vitamin A and metabolites of vitamin A that mediate the function of vitamin A, and includes for example all-trans RA (e.g. Sigma R2625), 9-cis RA (e.g. Sigma R4643), and retinol (e.g. Sigma R7632) as well as RA analogs (e.g. RAR agonists), such as AM580, a selective RARα agonist (Tocris 0760), AC55649, a selective RARβ agonist (Tocris 2436), and CD437, a selective RARγ agonist (Tocris 1549).

The terms "*TBX2*", *"MSX2"* and "*TBX3*" as used herein refer to the expression products of the T-box transcription factor 2 gene, the Msh Homeobox 2 gene and the T-box transcription factor 3 gene, respectively.

The term "pluripotent stem cell" (also referred to as "PSC") as used herein refers to a cell having the ability to differentiate to derivatives of all three germ layers, i.e., ectoderm, mesoderm and endoderm and also having self-renewal capacity which is an ability to maintain the pluripotency during cell division. "PSCs" include Embryonic Stem Cells (ESCs), which are derived from inner cell mass of blastocysts or morulae, including cells that have been serially passaged as cell lines. Embryonic stem cells, regardless of their source or the particular method used to produce them, can be identified based on their ability to differentiate into cells of all three germ layers, expression of at least Oct4 and alkaline phosphatase, and ability to produce teratomas when transplanted into immunodeficient animals. The term PSCs also includes induced PSCs (iPSCs), which are cells converted from somatic cells by a variety of methods, such as a transient overexpression of a set of transcription factors. A PSC may be a cell of any species with no limitation, and preferably a mammalian cell. It may be a rodent or primate cell. For example, it may be a monkey, mouse or a human pluripotent stem cell. The term "human pluripotent stem cells" or hPSCs includes human embryonic stem cells and human induced PSCs. Human embryonic stem cells may be obtained from established lines of human embryonic stem cells or human embryonic germ cells, such as, for example the human embryonic stem cell lines HI, H7, and H9 (WiCell).

The mesoderm cells, cardiomyocytes, cardiac progenitor cells, AVC-like cardiomyocytes and AVC cardiomyocytes according to the invention are preferably human.

The term "incubating" as used herein includes any *in vitro* method of maintaining and/or propagating a population of cells, including monolayer, bead, flask, or 3D cultures, optionally where ambient conditions are controlled as in an incubator and optionally involving passaging of cells. Steps that involve incubating the cells with one or more components, the components can be added simultaneously, at different times, for overlapping periods or for distinct periods. A factor can be added to the medium after the cells have started incubating in for example an induction medium or the factor can be added to the medium before the medium is added to the cells. Further, cells may be washed between incubations, for example to reduce the level of a component from a previous incubation.

The term "culturing" as used herein means any *in vitro* method of maintaining and propagating a population of cells at least through one cell division, including monolayer, bead, flask, or 3D cultures, optionally where ambient conditions are controlled as in an incubator.

The term " nodal signalling protein " as used herein refers to any molecule that activates Activin/Nodal signalling. Preferably, said molecule is Activin-A.

The term "TGFb superfamily protein" as used herein refers to any molecule that activates TGFb signalling. In some embodiments, an example of TGFb superfamily includes Activin A and any other related GFs/compounds. Preferably, said molecule is TGF-β.

The term "medium" as used herein refers to a culturing medium that is suitable for culturing animal cells, optionally supplemented with factors.

The term "early mesoderm induction medium" as used herein refers to a medium suitable for allowing a pluripotent stem cell to differentiate into a MESP1/2 mesoderm cell,
The term "late mesoderm induction medium" as used herein refers to a medium suitable for allowing a MESP1/2 mesoderm cell to differentiate into a ROR1+ and ROR2+ mesoderm cell.

The term "AVC cardiomyocyte induction medium" as used herein refers to a medium suitable for allowing an MESP1/2 mesoderm cells to differentiate into atrioventricular canal-like cardiomyocytes.

As used herein, the term "engineered tissue" refers to a tissue generated *in vitro* which comprises a group of cells, examples of which include organoid, assembloids, micro tissues, organ-on-chip or engineered heart tissues. A non-limiting functional characteristic of an engineered muscle tissue includes electrophysiological activity, such as an action potential, the ability to transmit an electrical signal to an adjacent cell, or biomechanical activity, such as contraction and/or the generation of force (which may be determined as described herein and in, for example, U.S. Pat. No. 8,492,150, U.S. Patent Publication Nos. 2011/0189719, 2012/0142556, and 2013/0046134, U.S. patent application Ser. No. 13/808,411, and PCT Publication No. WO 2013/086512. In an embodiment, an engineered tissue is an organoid.

As used herein, the term "organoid" is used to mean a 3-dimensional multicellular aggregate, preferably of mammalian cells in culture that recapitulates aspects of cellular organization and function of the heart. The term organoid also encompasses spheroids. In an embodiment, said engineered tissue is an assembloid.

"Assembloids" are 3-dimensional tissue constructs that result from the integration of multiple organoids or the combination of PSC-derived organoids with primary cell types or tissue explants. In the technical domain of the present disclosure, assembloids, may interchangeably be referred to by terms that are obvious alternatives thereof, examples of such terms include but are not limited to cardiac assembloids, cardiac microtissues, micro tissues, organ-on-chip or engineered heart tissues and engineered heart tissues.

As used herein, the term "comprising" when placed before the recitation of steps in a method means that the method encompasses one or more steps that are additional to those expressly recited, and that the additional one or more steps may be performed before, between, and/or after the recited steps. For example, a method comprising steps a, b, and c encompasses a method of steps a, b, x, and c, a method of steps a, b, c, and x, as well as a method of steps x, a, b, and c. Furthermore, the term "comprising" when placed before the recitation of steps in a method does not (although it may) require sequential performance of the listed steps, unless the content clearly dictates otherwise. For example, a method comprising steps a, b, and c encompasses, for example, a method of performing steps in the order of steps a, c, and b, the order of steps c, b, and a, and the order of steps c, a, and b, etc.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The suffix "(s)" at the end of any term in the present disclosure envisages in scope both the singular and plural forms of said term.

As used in this specification and the appended claims, the singular forms "a," "an" and "the" includes both singular and plural references unless the content clearly dictates otherwise. For example, the term "inserted at a position" as used herein in reference to a polypeptide sequence refers to insertion at one or more (such as one, two, three, etc.) amino acid positions in the polypeptide sequence. The use of the expression 'at least' or 'at least one' suggests the use of one or more elements or ingredients or quantities, as the use may be in the embodiment of the disclosure to achieve one or more of the desired objects or results. As such, the terms "a" (or "an"), "one or more", and "at least one" can be used interchangeably herein.

Numerical ranges stated in the form 'from x to y' include the values mentioned and those values that lie within the range of the respective measurement accuracy as known to the skilled person. If several preferred numerical ranges are stated in this form, of course, all the ranges formed by a combination of the different end points are also included.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

As used herein, the terms "include" (any form of "include", such as "include"), "have" (and "have"), "comprise" etc. any form of "having", "including" (and any form of "including" such as "including"), "containing", "comprising" or "comprises" are inclusive and will be understood to imply the inclusion of a stated element, integer or step, or group of elements, integers or steps, but not the exclusion of any other element, integer or step, or group of elements, integers or steps.

As regards the embodiments characterized in this specification, it is intended that each embodiment be read independently as well as in combination with another embodiment. For example, in case of an embodiment 1 reciting 3 alternatives A, B and C, an embodiment 2 reciting 3 alternatives D, E and F and an embodiment 3 reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

### Disclosure

Replicating the complexity of the heart through self-organization is challenging, given the myriad signaling cues and tight control of concentration gradients that are required in a temporal manner to generate tissues that are molecularly and functionally representative of the human heart. An alternative approach is to generate desired individual cell types using developmentally guided differentiation processes and subsequently assemble them into physiologically relevant tissues. The present disclosure sets out to provide such constructs to simulate the AV conduction axis, inspired by the assembloid approach reported to study neuronal interactions. The present disclosure shows that cardiac assembloids made by fusion of atrial, AVCM and ventricular cells or organoids reproduce electrical propagation patterns seen in the heart. This tissue model is reminiscent of the embryonic heart, where the AV canal provides electrical continuity between a singular atrial and singular ventricular chamber.1 Albeit being a simple approach, it has been demonstrated that it works reliably.

With the above context, various aspects of the present disclosure have been explained in detail below.

### Generation of AVC-like cell(s) from cardiogenic mesoderm cells

It is an object of the present disclosure to provide a method for the generation of atrioventricular canal (AVC) cardiomyocyte-like cells from pluripotent stem cells or mesoderm cells.

In view of the same, the present disclosure provides a method of generating AVC cardiomyocyte -like cell(s) from cardiogenic mesoderm cells comprising:
incubating the cardiogenic mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2, TBX2.*

In some embodiments, the AVC cardiomyocyte induction medium is any known AVC cardiomyocyte induction medium supplemented with WNT2 and RA.

In some embodiments, the AVC cardiomyocyte induction medium is BPEL, wherein in the aforesaid method, the BPEL further comprises WNT2 and RA.

In some embodiments, the AVC cardiomyocyte induction medium comprises about 1 ng/mL to about 20 ng/mL WNT2.

In some embodiments, the AVC cardiomyocyte induction medium comprises about 1 ng/mL to about 5 ng/mL, about 5 ng/mL to about 10 ng/mL, about 10 ng/mL to about 20 ng/mL, about 1 ng/mL, about 2 ng/mL, 3 ng/mL, about 4 ng/mL, about 5 ng/mL, about 6 ng/mL, about 7 ng/mL, about 8 ng/mL, about 10 ng/mL, about 11 ng/mL, about 12 ng/mL, about 13 ng/mL, about 14 ng/mL, about 15 ng/mL, about 16 ng/mL, about 17 ng/mL, about 18 ng/mL, about 19 ng/mL or about 20 ng/mL of WNT2.

In a preferred embodiment, the AVC cardiomyocyte induction medium comprises about 5 ng/mL WNT2.

In some embodiments, the AVC cardiomyocyte induction medium comprises about 50 nM to about 1000 nM RA.

In some embodiments, the AVC cardiomyocyte induction medium comprises about 50 nM to about 200 nM, about 200 nM to about 400 nM, about 400 nM to about 600 nM, about 600 nM to about 800 nM, about 800 nM to about 1000 nM, about 50 nM, about 100 nM, about 200 nM, about 300 nM, about 400 nM, about 500 nM, about 600 nM, about 700 nM, about 800 nM, about 900 nM or about 1000 nM RA.

In a preferred embodiment, the AVC cardiomyocyte induction medium comprises about 250 nM RA.

In some embodiments, the AVC cardiomyocyte induction medium comprises about 1 ng/mL to about 20 ng/mL WNT2 and about 50 nM to about 1000 nM RA.

In some embodiments, the AVC cardiomyocyte induction medium comprises about 3 ng/mL to about 10 ng/mL WNT2 and about 200 nM to about 300 nM RA.

In a preferred embodiment, the AVC cardiomyocyte induction medium comprises about 5 ng/mL WNT2 and about 250 nM RA.

In some embodiments, the AVC cardiomyocyte induction medium may further comprise a BMP ligand such as but not limited to *BMP4* and *EMP2.* In some embodiments, the BMP ligand may be incorporated at a concentration of about 1 - 20 ng/mL.

Thus, in some embodiments, the method of generating AVC cardiomyocyte -like cell(s) from cardiogenic mesoderm cells comprises:
incubating the cardiogenic mesoderm cells in an AVC cardiomyocyte induction medium comprising WNT2, retinoic acid (RA) and optionally, a BMP ligand such as but not limited to *BMP4* and/or *BMP2* to obtain the AVC-like cell(s), wherein the AVC-like cell(s) express *TBX3* and at least one of *BMP2, MSX2, TBX2.*

In some embodiments, the cardiogenic mesoderm cells are incubated in the AVC cardiomyocyte induction medium comprising WNT2, retinoic acid (RA) and optionally, a BMP ligand such as but not limited to *BMP4* and/or *BMP2* for about 24 hours to about 96 hours.

In some embodiments, the cardiogenic mesoderm cells are incubated in the AVC cardiomyocyte induction comprising WNT2, retinoic acid (RA) and optionally, a BMP ligand such as but not limited to *BMP4* and/or *BMP2* for about 24 hours to about 60 hours, about 60 hours to about 72 hours, about 72 hours to about 96 hours, about 24 hours, about 36 hours, about 48 hours, about 56 hours, about 68 hours, about 72 hours, about 84 hours or about 96 hours.

In a preferred embodiment, the cardiogenic mesoderm cells are incubated in the AVC cardiomyocyte induction medium comprising WNT2, retinoic acid (RA) and optionally, a BMP ligand such as but not limited to *BMP4* and/or *BMP2* for about 72 hours.

Thus, in a preferred embodiment the method of generating AVC-like cell(s) from cardiogenic mesoderm cells comprises:
incubating the cardiogenic mesoderm cells in an AVC cardiomyocyte induction medium comprising about 1 ng/mL to about 20 ng/mL WNT2 and about 50 nM to about 1000 nM RA for about 24 hours to about 96 hours to obtain the AVC-like cell(s), wherein the AVC-like cell(s) express *TBX3* and at least one of *BMP2, MSX2, TBX2.*

In some embodiments, the method of generating AVC-like cell(s) from cardiogenic mesoderm cells comprises:
incubating the cardiogenic mesoderm cells in an AVC cardiomyocyte induction medium comprising about 1 ng/mL to about 20 ng/mL WNT2, about 50 nM to about 1000 nM RA and optionally, a BMP ligand such as but not limited to *BMP4* and/or *BMP2* for about 24 hours to about 96 hours to obtain the AVC-like cell(s), wherein the AVC-like cell(s) express *TBX3* and at least one of *BMP2, MSX2, TBX2.*

In another preferred embodiment the method of generating AVC-like cell(s) from cardiogenic mesoderm cells comprises:
incubating the cardiogenic mesoderm cells in an AVC cardiomyocyte induction medium comprising about 5 ng/mL WNT2 and about 250 nM RA for about 72 hours to obtain the AVC-like cell(s), wherein the AVC-like cell(s) express *TBX3* and at least one of *BMP2, MSX2* and *TBX2.*

In some embodiments, the obtained AVC-like cardiomyocytes express *TBX3* and *BMP2.*

In some embodiments, the obtained AVC-like cardiomyocytes express *TBX3* and *MSX2.*

In some embodiments, the obtained AVC-like cardiomyocytes express *TBX3* and *TBX2.*

In some embodiments, the obtained AVC-like cardiomyocytes express *TBX3* and at least two of *BMP2, MSX2* and *TBX2.*

In some embodiments, the obtained AVC-like cardiomyocytes express *TBX3, BMP2* and *MSX2.*

In some embodiments, the obtained AVC-like cardiomyocytes express *TBX3, BMP2* and *TBX2.*

In some embodiments, the obtained AVC-like cardiomyocytes express *TBX3, MSX2* and *TBX2.*

In some embodiments, the obtained AVC-like cardiomyocytes express *TBX3, BMP2, MSX2* and *TBX2.*

In some embodiments, the obtained AVC-like cardiomyocytes exhibit electrophysiological properties of AVC cardiomyocytes.

In some embodiments, the said electrophysiological properties include exhibition of HCN dependent funny current, I_{f} or response to ivabradine, which targets I_{f} and response to carbachol, which activates inward rectifier potassium current, I_{KACH}.

In some embodiments, the electrophysiological properties exhibited by AVC-like cardiomyocytes comprise exhibition of HCN dependent funny current.

Without intending to be limited by theory, the treatment of cardiac mesoderm cells with WNT2 and retinoic acid (RA) yields a cardiomyocyte population that preferentially expressed transcription factors *BMP2, MSX2, TBX2,* and *TBX3,* which are known to steer AVC development. As demonstrated in the examples section, molecular characterization of WNT2 and RA treated cardiomyocytes reveals that the majority of cells from this group identified with AVCM of the mouse heart. Ion channel expression, action potential properties, I_{f} and I_{Na} measurements are also in line with expected behavior of AV cardiomyocytes. In a non-limiting embodiment, conduction velocity of the AVC-like cardiomyocytes obtained from the above described method ranges from about 1.5 cm/sec to about 1.8 cm/sec, which is comparable to human fetal AVN, reported to be about 1 cm/sec.

### The cardiogenic mesoderm cells

In some embodiments, the cardiogenic mesoderm cells employed in the above defined method express at least one of MESP1 and MESP2, and hereinafter referred to as MESP1/2 mesoderm cells.

In some embodiments, the MESP1/2 mesoderm cells express MESP1.

In some embodiments, the MESP1/2 mesoderm cells express MESP2.

In some embodiments, the MESP1/2 mesoderm cells express MESP1 and MESP2.

In some embodiments, the MESP1/2 mesoderm cells may optionally further express EOMES. Thus, in some embodiments, the MESP1/2 mesoderm cells express EOMES and at least one of MESP1 and MESP2.

In some embodiments, the MESP1/2 mesoderm cells express MESP1 and EOMES.

In some embodiments, the MESP1/2 mesoderm cells express MESP2 and EOMES.

In some embodiments, the MESP1/2 mesoderm cells express MESP1, MESP2 and EOMES.

Accordingly, the present disclosure provides a method of generating AVC-like cell(s) from cardiogenic mesoderm cells comprising:
incubating MESP1/2 mesoderm cells in an AVC cardiomyocyte induction medium comprising WNT2, retinoic acid (RA) and optionally, a BMP ligand such as but not limited to *BMP4* to obtain the AVC-like cell(s), wherein the AVC-like cell(s) express *TBX3* at least one of *BMP2, MSX2* and *TBX2.*

In some embodiments, the method of generating AVC-like cell(s) from cardiogenic mesoderm cells comprises:
incubating MESP1/2 mesoderm cells in an AVC cardiomyocyte induction medium comprising WNT2 and retinoic acid (RA) to obtain the AVC-like cell(s), wherein the AVC-like cell(s) express *TBX3* at least one of *BMP2, MSX2* and *TBX2.*

### Generation of the MESP1/2 mesoderm cells

In some embodiments, the above described MESP1/2 mesoderm cells may be commercially procured MESP1/2 mesoderm cells or the MESP1/2 mesoderm cells may be synthesized.

As mentioned above, in some embodiments, the MESP1/2 mesoderm cells may express at least one of MESP1 and MESP2 and optionally, EOMES. In some embodiments, the MESP1/2 mesoderm cells express MESP1 and EOMES. In some embodiments, the MESP1/2 mesoderm cells express MESP2 and EOMES. In some embodiments, the MESP1/2 mesoderm cells express MESP1, MESP2 and EOMES.

The MESP1/2 mesoderm cells may be generated using methods known in the art.

In some embodiments, the MESP1/2 mesoderm cells are obtained from pluripotent stem cell (PSCs).

In some embodiments, the MESP1/2 mesoderm cells are prepared by incubating a pluripotent stem cell (PSC) in an early mesoderm induction medium comprising one or more of a BMP ligand, a nodal signalling protein and activators of WNT signalling to generate MESP1/2 mesoderm cells expressing MESP1 and/or MESP2 and optionally, EOMES.

In some embodiments, a non-limiting example of the nodal signalling protein includes but it not limited to a TGFb signalling protein. Another non-limiting example of the said protein include Activin-A.

Thus, in some preferred embodiments, the MESP1/2 mesoderm cells are prepared by incubating a pluripotent stem cell (PSC) in an early mesoderm induction medium comprising one or more of a BMP ligand, a TGFb superfamily protein and activators of WNT signalling to generate MESP1/2 mesoderm cells expressing MESP1 and/orMESP2 and optionally, EOMES.

Accordingly, in some embodiments, the MESP1/2 mesoderm cell is prepared by incubating pluripotent stem cells (PSCs) in an early mesoderm induction medium comprising an activator of WNT signalling and optionally, a BMP ligand and/or a TGFb signalling protein to generate the MESP1/2 mesoderm cells; wherein the MESP1/2 mesoderm cell expresses at least one of MESP1 and MESP2 and optionally, EOMES.

In some embodiments, the MESP1/2 mesoderm cell is prepared by incubating pluripotent stem cells (PSCs) in an early mesoderm induction medium comprising an activator of WNT signalling and a BMP ligand and/or a TGFb superfamily protein for a sufficient period of time to generate the MESP1/2 mesoderm cells; wherein the MESP1/2 mesoderm cell expresses at least one of MESP1 and MESP2, and optionally, EOMES.

In some embodiments, the MESP1/2 mesoderm cell is prepared by incubating pluripotent stem cells (PSCs) in an early mesoderm induction medium comprising an activator of WNT signalling and a BMP ligand for a sufficient period of time to generate the MESP1/2 mesoderm cells; wherein the MESP1/2 mesoderm cell expresses at least one of MESP1 and MESP2, and optionally, EOMES.

In some embodiments, the MESP1/2 mesoderm cell is prepared by incubating pluripotent stem cells (PSCs) in an early mesoderm induction medium comprising an activator of WNT signalling and a TGFb superfamily protein for a sufficient period of time to generate the MESP1/2 mesoderm cells; wherein the MESP1/2 mesoderm cell expresses at least one of MESP1 and MESP2, and optionally, EOMES.

In some embodiments, the MESP1/2 mesoderm cell is prepared by incubating pluripotent stem cells (PSCs) in an early mesoderm induction medium comprising an activator of WNT signalling, a BMP ligand and a TGFb signalling protein for a sufficient period of time to generate the MESP1/2 mesoderm cells; wherein the MESP1/2 mesoderm cell expresses at least one of MESP1 and MESP2, and optionally, EOMES.

The early mesoderm induction medium may be any medium that is routinely used for culturing animal cells can be used, except that preferably no growth factors or serum are present or are added to the media. In some embodiments, the early mesoderm induction medium is selected from a group comprising APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media. In some embodiments, non-growth factor additives, such as antibiotics, B-27 supplement (with or without insulin), amino acids, salts, ascorbic acid and thioglycerol may be added to the said early mesoderm induction medium.

In a non-limiting, preferred embodiment, the early mesoderm induction medium comprises about 20 ng/mL Activin-A, about 20 ng/mL *BMP4,* and about 1.5 µmol/L. CHIR99021. More preferably, said early mesoderm induction medium may comprise BPEL medium supplemented with about 20 ng/mL Activin-A, about 20 ng/mL *BMP4* and about 1.5 µmol/L CHIR99021.

In a non-limiting embodiment, the PSCs may be seeded at a density of about 2.5-3×10⁴ cells/cm² and differentiation toward early mesoderm may be induced or triggered when cells reach about 80% to about 90% confluency.

In some embodiments, the pluripotent stem cells (PSCs) are incubated in the early mesoderm induction medium comprising an activator of WNT signalling and a BMP ligand and/or a TGFb superfamily protein for about 2 days to about 6 days to generate the MESP1/2 mesoderm cells.

In some embodiments, the pluripotent stem cells (PSCs) are incubated in the early mesoderm induction medium comprising an activator of WNT signalling and a BMP ligand and/or a TGFb superfamily protein for about 2 days to about 4 days, about 4 days to about 6 days, about 2 days, about 3 days, about 4 days, about 5 days or about 6 days to generate the MESP1/2 mesoderm cells.

In a preferred embodiment, the pluripotent stem cells (PSCs) are incubated in the early mesoderm induction medium comprising an activator of WNT signalling and a BMP ligand and/or a TGFb superfamily protein for about 6 days to generate the MESP1/2 mesoderm cells.

In some embodiments, obtained MESP1/2 mesoderm cells express MESP1 and/or MESP2 and optionally, EOMES.

In some embodiments, obtained MESP1/2 mesoderm cells express MESP1.

In some embodiments, obtained MESP1/2 mesoderm cells express MESP2.

In some embodiments, obtained MESP1/2 mesoderm cells express MESP1 and MESP2.

In some embodiments, obtained MESP1/2 mesoderm cells express MESP1 and EOMES.

In some embodiments, obtained MESP1/2 mesoderm cells express MESP2 and EOMES.

In some embodiments, obtained MESP1/2 mesoderm cells express MESP1, MESP2 and EOMES.

Put together, in some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein to generate MESP1/2 mesoderm cells; and
incubating the MESP1/2 mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein for about 2 days to about 6 days to generate MESP1/2 mesoderm cells; and
incubating the MESP1/2 mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising BPEL medium supplemented with about 20 ng/mL Activin-A, about 20 ng/mL *BMP4* and about 1.5 µmol/LCHIR99021 for about 2 days to about 6 days to generate MESP1/2 mesoderm cells; and
incubating the MESP1/2 mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

### ROR1+ and ROR2+ MESP1/2 mesoderm cells and generation thereof

In some embodiments, the cardiogenic mesoderm cells employed in the above defined method of generating the AVC-like cell(s) are MESP1/2 mesoderm cells that express the ROR-family receptor tyrosine kinases.

In some embodiments, the ROR-family receptor tyrosine kinases are ROR1 and ROR2, wherein MESP1/2 mesoderm cells expressing ROR1 and ROR2 are hereinafter interchangeably referred to as ROR1+ and ROR2+ MESP1/2 mesoderm cells.

Accordingly, in some embodiments, the cardiogenic mesoderm cells employed in the above defined method of generating the AVC-like cell(s) are ROR1+ and ROR2+ MESP1/2 mesoderm cells.

Thus, in some embodiments, the method of generating AVC-like cell(s) from cardiogenic mesoderm cells comprises:
incubating ROR1+ and ROR2+ MESP1/2 mesoderm cells in an AVC cardiomyocyte induction medium comprising WNT2 and retinoic acid (RA) to obtain the AVC-like cell(s), wherein the AVC-like cell(s) express *TBX3* at least one of *BMP2, MSX2* and *TBX2.*

In some embodiments, the ROR1+ and ROR2+ MESP1/2 mesoderm cells are obtained by:
incubating MESP1/2 mesoderm cells in a late mesoderm induction medium comprising a WNT inhibitor to generate the ROR1+ and ROR2+ mesoderm cells.

In some embodiments, the late mesoderm induction medium may be any medium that is routinely used for culturing animal cells, except that preferably no growth factors or serum are present or are added to the media. Non-limiting examples of such late mesoderm induction media include APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media. In some embodiments, non-growth factor additives, such as but not limited to antibiotics, B-27 supplement (with or without insulin), amino acids, salts, ascorbic acid and thioglycerol may be added to the late mesoderm induction medium. In the context of the present disclosure, the late mesoderm induction medium further comprises a WNT inhibitor.

The presence of a WNT inhibitor in the late mesoderm induction medium steers the MESP1/2 mesoderm cells toward ROR1+ and ROR2+ mesoderm cells.

In some embodiments, the WNT inhibitor is selected from a group comprising XAV939, IWP 2, and iCRT14 or any combination thereof.

In a preferred embodiment, the WNT inhibitor is XAV939.

In an embodiment, the late mesoderm induction medium comprises the WNT inhibitor at a concentration of about 200 nM to about 10 µM.

In some embodiments, the late mesoderm induction medium comprises the WNT inhibitor at a concentration of about 200 nM to about 500 nM, about 500 nM to about 1 µM , about 1 µM to about 4 µM, about 4 µM to about 8 µM , about 8 µM to about 10 µM, about 200 nM, about 500 nM, about 800 nM, about 1 µM, about 1.5 µM, about 2 µM, about 2.5 µM, about 3 µM, about 3.5 µM, about 4 µM, about 4.5 µM, about 5 µM, about 5.5 µM, about 6 µM, about 6.5 µM, about 7 µM, about 7.5 µM, about 8 µM, about 8.5 µM, about 9 µM, about 9.5 µM or about 10 µM.

In a preferred embodiment, the late mesoderm induction medium comprises about 200 nM to about 8 µM or about 300 nM to about 6 µM of the WNT inhibitor.

In another preferred embodiment, the late mesoderm induction medium comprises about 1 µM, about 2 µM, about 3 µM, about 4 µM, about 5 µM, about 8 µM, or up to about 10 µM of XAV939.

In some embodiments, the MESP1/2 mesoderm cells are incubated in the late mesoderm induction medium comprising the WNT inhibitor for about 18 hours to about 30 hours to generate the ROR1+ and ROR2+ mesoderm cells.

In some embodiments, the MESP1/2 mesoderm cells are incubated in the late mesoderm induction medium comprising the WNT inhibitor for about 18 hours to about 25 hours, about 25 hours to about 30 hours, about 18 hours, about 19 hours, about 20 hours, about 21 hours, about 22 hours, about 23 hours, about 24 hours, about 25 hours, about 26 hours, about 27 hours, about 28 hours, about 29 hours or about 30 hours to generate the MESP1/2 mesoderm cells to generate the ROR1+ and ROR2+ mesoderm cell.

In a preferred embodiment, the MESP1/2 mesoderm cells are incubated in the late mesoderm induction medium comprising the WNT inhibitor for about 24 hours to generate the ROR1+ and ROR2+ mesoderm cell.

In yet another preferred embodiment, the MESP1/2 mesoderm cells may be incubated with BPEL medium containing about 5 µM XAV939 for about 24 hours to steer the MESP1/2 mesoderm cells towards ROR1+ and ROR2+ mesoderm cells.

In a non-limiting embodiment, the aforesaid method of generating the ROR1+ and ROR2+ mesoderm cells may further comprise a cell enrichment step to increase the number of said ROR1+ and ROR2+ mesoderm cells. Such methods are well known in the art and include but are not limited to for instance immunomagnetic cell separation, fluorescence-activated cell sorting (FACS), density gradient centrifugation, immunodensity cell isolation and microfluidic cell sorting.

Accordingly, in some embodiments, the ROR1+ and ROR2+ mesoderm cells are obtained by: incubating MESP1/2 mesoderm cells in a late mesoderm induction medium comprising a WNT inhibitor to generate the ROR1+ and ROR2+ mesoderm cells; and
optionally, subjecting the cells obtained after the incubation to cell enrichment to increase the number of said ROR1+ and ROR2+ mesoderm cells.

In some embodiments, the ROR1+ and ROR2+ mesoderm cells are obtained by:
incubating MESP1/2 mesoderm cells in a late mesoderm induction medium comprising a WNT inhibitor to generate the ROR1+ and ROR2+ mesoderm cells; and
subjecting the cells obtained after the incubation to cell enrichment to increase the number of said ROR1+ and ROR2+ mesoderm cells.

In some embodiments, the ROR1+ and ROR2+ mesoderm cells are obtained by:
incubating MESP1/2 mesoderm cells in a late mesoderm induction medium comprising a WNT inhibitor to generate the ROR1+ and ROR2+ mesoderm cells; and
subjecting the cells obtained after the incubation to cell enrichment to increase the number of said ROR1+ and ROR2+ mesoderm cells; wherein the cell enrichment is performed by technique(s) selection from a group comprising immunomagnetic cell separation, fluorescence-activated cell sorting (FACS), density gradient centrifugation, immunodensity cell isolation and microfluidic cell sorting.

In some embodiments, the present disclosure provides a method of generating AVC-like cell(s) from cardiogenic mesoderm cells comprising:
incubating MESP1/2 mesoderm cells in a late mesoderm induction medium comprising a WNT inhibitor to generate the ROR1+ and ROR2+ mesoderm cells;
optionally, subjecting the cells obtained after the incubation to cell enrichment to increase the number of said ROR1+ and ROR2+ mesoderm cells; and
incubating the ROR1+ and ROR2+ mesoderm cells in an AVC cardiomyocyte (AVC) induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating MESP1/2 mesoderm cells in a late mesoderm induction medium comprising WNT inhibitor XAV939 to generate the ROR1+ and ROR2+ mesoderm cells; and
incubating the ROR1+ and ROR2+ mesoderm cells in AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating MESP1/2 mesoderm cells in a late mesoderm induction medium comprising about 200 nM to about 10 µM of WNT inhibitor XAV939 to generate the ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating AVC cardiomyocyte like cell(s) from MESP1/2 mesoderm cells comprises:
incubating MESP1/2 mesoderm cells in a late mesoderm induction medium comprising WNT inhibitor XAV939 to generate the ROR1+ and ROR2+ mesoderm cells; and
incubating the ROR1+ and ROR2+ mesoderm cells in an AVC cardiomyocyte induction medium comprising about 1 ng/mL to about 20 ng/mL WNT2 and about 50 nM to about 1000 nM RA obtain the AVC-like cell(s), wherein the AVC-like cell(s) express *TBX3* and at least one of *BMP2, MSX2* and *TBX2.*

In some embodiments, the method of generating the AVC-like cell(s) from MESP1/2 mesoderm cells comprises:
incubating MESP1/2 mesoderm cells in a late mesoderm induction medium comprising a WNT inhibitor for about 18 hours to about 30 hours, preferably about 24 hours to generate the ROR1+ and ROR2+ mesoderm cells; and
incubating the ROR1+ and ROR2+ mesoderm cells in an AVC cardiomyocyte induction medium comprising WNT2 and retinoic acid (RA) for about 24 hours to about 96 hours, preferably about 72 hours to obtain the AVC-like cell(s), wherein the AVC-like cell(s) express *TBX3* and at least one of *BMP2, MSX2* and *TBX2.*

In some embodiments, the method of generating AVC-like cell(s) from MESP1/2 mesoderm cells comprises:
incubating MESP1/2 mesoderm cells in a late mesoderm induction medium comprising about 200 nM to about 10 µM WNT inhibitor XAV939 for about 18 hours to about 30 hours, preferably about 24 hours to generate the ROR1+ and ROR2+ mesoderm cells; and
incubating the ROR1+ and ROR2+ mesoderm cells in an AVC cardiomyocyte induction medium comprising about 1 ng/mL to about 20 ng/mL WNT2 and about 50 nM to about 1000 nM RA for about 60 hours to about 80 hours to obtain the AVC-like cell(s), wherein the AVC-like cell(s) express *TBX3* and at least one of *BMP2, MSX2* and *TBX2.*

Without intending to be limited by theory, incubation conditions for cell cultures are known in the art. For example, the conditions typically include culturing at a temperature of between about 32°C to about 40 °C, for example, about 32°C, about 33°C, about 34°C, about 35°C, about 36°C, about 37°C, about 38°C, about 39°C or about 40 °C, preferably at about 37 °C. The CO₂ concentration may range between about 1% to about 10%, for example, about 2% to about 7%, or about 5% or any range or value between about 1% and 10%. In some embodiments, the oxygen tension is adjusted to generally provide normoxic conditions and is preferably about 20%.

Put together, in some embodiments, the method of generating the AVC-like cell(s) from cardiogenic mesoderm cells comprises:
incubating pluripotent stem cells (PSCs) in an early mesoderm induction medium comprising one or more of a BMP ligand, a TGFb superfamily protein and activators of WNT signalling to generate MESP1/2 mesoderm cells;
incubating the MESP1/2 mesoderm cells in a late mesoderm induction medium comprising an WNT inhibitor to generate the ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein to generate MESP1/2 mesoderm cells;
incubating the MESP1/2 mesoderm cells in a late mesoderm induction medium comprising WNT inhibitor XAV939 to generate the ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein to generate MESP1/2 mesoderm cells;
incubating the MESP1/2 mesoderm cells in a late mesoderm induction medium comprising about 200 nM to about 10 µM of WNT inhibitor XAV939 to generate the ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein to generate MESP1/2 mesoderm cells;
incubating the MESP1/2 mesoderm cells in a late mesoderm induction medium comprising WNT inhibitor XAV939 to generate the ROR1+ and ROR2+ mesoderm cells; and
incubating the ROR1+ and ROR2+ mesoderm cells in an AVC cardiomyocyte induction medium comprising about 3 ng/mL to about 10 ng/mL WNT2 and about 200 nM to about 300 nM RA to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising an activator of WNT signalling and a BMP ligand and/or a TGFb signalling protein for about 2 days to about 6 days to generate MESP1/2 mesoderm cells;
incubating MESP1/2 mesoderm cells in a late mesoderm induction medium comprising a WNT inhibitor for about 18 hours to about 30 hours, preferably about 24 hours to generate the ROR1+ and ROR2+ mesoderm cells; and
incubating the ROR1+ and ROR2+ mesoderm cells in an AVC cardiomyocyte induction medium comprising WNT2 and retinoic acid (RA) for about 60 hours to about 80 hours, preferably about 72 hours to obtain the AVC-like cell(s), wherein the AVC-like cell(s) express *TBX3* and at least one of *BMP2, MSX2* and *TBX2.*

In some embodiments, the method of generating the AVC-like cell(s) from cardiogenic mesoderm cells comprises:
incubating pluripotent stem cells (PSCs) in an early mesoderm induction medium comprising BPEL medium supplemented with about 20 ng/mL Activin-A, about 20 ng/mL *BMP4* and about 1.5 µmol/LCHIR99021 for about 2 days to about 6 days to generate MESP1/2 mesoderm cells;
incubating the MESP1/2 mesoderm cells in a late mesoderm induction medium comprising about 200 nM to about 10 µM WNT inhibitor XAV939 for about 18 hours to about 30 hours, preferably about 24 hours to generate the ROR1+ and ROR2+ mesoderm cells; and
incubating the ROR1+ and ROR2+ mesoderm cells in an AVC cardiomyocyte induction medium comprising about 3 ng/mL to about 10 ng/mL WNT2 and about 200 nM to about 300 nM RA for about 60 hours to about 80 hours, preferably about 72 hours to obtain the AVC-like cell(s), wherein the AVC-like cell(s) express *TBX3* and at least one of *BMP2, MSX2* and *TBX2.*

### Media employed in the above methods:

In some embodiments, the present disclosure further provides the early mesoderm induction medium, the late mesoderm induction medium and the AVC cardiomyocyte induction medium employed in the aforesaid method(s).

In some embodiments, provided herein is an early mesoderm induction medium comprising one or more of a BMP ligand, a TGFb superfamily protein and activators of WNT signalling, wherein the early mesoderm induction medium may find application in generating MSP1 mesoderm cells from pluripotent stem cells (PSCs).

In some embodiments, a non-limiting example of the nodal signalling protein is a TGFb signalling protein.

Thus, in some embodiments, the early mesoderm induction medium comprises one or more of a BMP ligand, a TGFb superfamily protein and activators of WNT signalling, wherein the early mesoderm induction medium may find application in generating MSP1 mesoderm cells from pluripotent stem cells (PSCs).

In some embodiments, the early mesoderm induction medium is selected from a group comprising APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media. In some embodiments, non-growth factor additives, such as antibiotics, B-27 supplement (with or without insulin), amino acids, salts, ascorbic acid, and thioglycerol may be added to the said early mesoderm induction medium.

In a preferred embodiment, the early mesoderm induction medium comprises about 20 ng/mL Activin-A, about 20 ng/mL *BMP4,* and about 1.5 µmol/L. CHIR99021. More preferably, said early mesoderm induction medium may comprise BPEL medium supplemented with about 20 ng/mL Activin-A, about 20 ng/mL *BMP4* and about 1.5 µmol/L. CHIR99021.

In some embodiments, the present invention provides an early mesoderm induction medium comprising one or more of a BMP ligand, a TGFb superfamily protein and activators of WNT signalling for use in generating MSP1 mesoderm cells from pluripotent stem cells (PSCs).

The present disclosure further provides a late mesoderm induction medium comprising a WNT inhibitor, wherein the said late mesoderm induction medium may find application in generating ROR1+ and ROR2+ mesoderm cells from MESP1/2 mesoderm cells.

In some embodiments, examples of such late mesoderm induction media include APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media. In some embodiments, non-growth factor additives, such as but not limited to antibiotics, B-27 supplement (with or without insulin), amino acids, salts, ascorbic acid and thioglycerol may be added to the late mesoderm induction medium. The late mesoderm induction medium further comprises a WNT inhibitor.

In some embodiments, the WNT inhibitor is selected from a group comprising XAV939, IWP 2, and iCRT14 or any combination thereof.

In a preferred embodiment, the WNT inhibitor is XAV939.

In an embodiment, the late mesoderm induction medium comprises the WNT inhibitor at a concentration of about 200 nM to about 10 µM.

In some embodiments, the present invention provides a late mesoderm induction medium comprising a WNT inhibitor for use in generating ROR1+ and ROR2+ mesoderm cells from MESP1/2 mesoderm cells.

The present disclosure further provides an AVC cardiomyocyte induction medium comprising WNT2 and retinoic acid (RA), wherein the AVC cardiomyocyte induction medium may find application in generating AVC-like cell(s) from ROR1+ and ROR2+ MESP1/2 mesoderm cells.

In some embodiments, the AVC cardiomyocyte induction medium is any known AVC cardiomyocyte induction medium supplemented with WNT2 and RA.

In some embodiments, the AVC cardiomyocyte induction medium is BPEL, wherein in the aforesaid method, the BPEL further comprises WNT2 and RA.

In some embodiments, the AVC cardiomyocyte induction medium comprises about 3 ng/mL to about 10 ng/mL WNT2 and about 200 nM to about 300 nM RA.

In a preferred embodiment, the AVC cardiomyocyte induction medium comprises about 5 ng/mL WNT2 and about 250 nM RA.

In some embodiments, the AVC cardiomyocyte induction medium may further comprise a BMP ligand such as but not limited to *BMP4* and/or *BMP2.*

Thus, in some embodiments, the present disclosure provides an AVC cardiomyocyte induction medium comprising WNT2, retinoic acid (RA) and optionally, BMP ligand such as but not limited to *BMP4* and/or *BMP2,* wherein the AVC cardiomyocyte induction medium may find application in generating AVC-like cell(s) from ROR1+ and ROR2+ MESP1/2 mesoderm cells.

Molecular and functional characterization described in the working examples also demonstrate the translatability of WNT2 and RA driven AVCM differentiation to an independent hiPSC line.

### AVC-like cell(s)

The present disclosure further provides AVC-like cell(s) obtainable by the method as described above.

Accordingly, envisaged herein are AVC-like cell(s) obtainable by a method comprising: incubating the cardiogenic mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, envisaged herein are AVC-like cell(s) obtainable by a method comprising:
incubating MESP1/2 mesoderm cells in a late mesoderm induction medium comprising a WNT inhibitor to generate the ROR1+ and ROR2+ mesoderm cells;
optionally, subjecting the cells obtained after the incubation to cell enrichment to increase the number of said ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, envisaged herein are AVC-like cell(s) obtainable by a method comprising:
incubating pluripotent stem cells (PSCs) in an early mesoderm induction medium comprising an activator of WNT signalling and a BMP ligand or a TGFb signalling protein to generate MESP1/2 mesoderm cells;
incubating the MESP1/2 mesoderm cells in a late mesoderm induction medium comprising an WNT inhibitor to generate the ROR1+ and ROR2+ mesoderm cells; and
incubating the ROR1+ and ROR2+ mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

In some embodiments, further envisaged herein are AVC-like cell(s) or a population of AVC-like cell(s) expressing *TBX3* and at least one of *BMP2, MSX2* and *TBX2.*

In some embodiments, the AVC-like cell(s) or the population of AVC-like cell(s) express *TBX3* and at least two of *TBX2, MSX2* and *TBX3.*

In some embodiments, the AVC-like cell(s) or the population of AVC-like cell(s) express *TBX3* and *EMP2.*

In some embodiments, the AVC-like cell(s) or the population of AVC-like cell(s) express *TBX3* and *MSX2.*

In some embodiments, the AVC-like cell(s) or the population of AVC-like cell(s) express *TBX3* and *TBX2.*

In some embodiments, the AVC-like cell(s) or the population of AVC-like cell(s) express *TBX3* and at least two of *BMP2, MSX2* and *TBX2.*

In some embodiments, the AVC-like cell(s) or the population of AVC-like cell(s) express *TBX3, BMP2* and *MSX2.*

In some embodiments, the AVC-like cell(s) or the population of AVC-like cell(s) express *TBX3, BMP2* and *TBX2.*

In some embodiments, the AVC-like cell(s) or the population of AVC-like cell(s) express *TBX3, MSX2* and *TBX2.*

In some embodiments, the AVC-like cell(s) or the population of AVC-like cell(s) express *TBX3, BMP2, MSX2* and *TBX2.*

In some embodiments, the AVC-like cell(s) or the population of AVC-like cell(s) exhibit one or more electrophysiological properties of AVC cardiomyocytes.

In some embodiments, the said one or more electrophysiological properties include but are not limited to responses to ivabradine, which targets funny current, I_{f} or exhibition of HCN dependent funny current and response to carbachol, which activates inward rectifier potassium current, I_{KACH}.

In some embodiments, the electrophysiological properties exhibited by AVC-like cell(s) or the population of AVC-like cell(s) comprise exhibition of HCN dependent funny current.

In some embodiments, the AVC-like cell(s) bear molecular identity to *in vivo* AVCM.

In a non-limiting embodiment, the conduction velocity of the AVC-like cells ranges from about 1.5 cm/sec to about 1.8 cm/sec, comparable to human fetal AVN, reported to be about 1 cm/sec.

Without intending to be limited by theory, notable electrophysiological properties of AVC-like cells (AVCM) in comparison with ventricular-like cardiomyocytes (VCM) include a) shorter cycle length, and b) slower upstroke velocity. In a non-limiting embodiment, cycle lengths in AVCM are shorter compared with VCM.

### Engineered tissue

In some embodiments, the present disclosure provides an engineered tissue comprising the comprising the AVC-like cardiomyocyte(s) of the present disclosure.

In some embodiments, the engineered tissue may further comprise one or more of Sinoatrial nodal cardiomyocytes (SANCM), Atrial cardiomyocytes (ACM) and Ventricular cardiomyocytes (VCM).

In some embodiments, the engineered tissue may further comprise ACM and/or VCM cardiomyocytes.

In some embodiments, the engineered tissue is an organoid. Accordingly, in some embodiments, the engineered tissue is an organoid comprising the AVC-like cardiomyocyte(s) of the present disclosure (AVCM), ACM and/or VCM cardiomyocyte(s).

In some embodiments, the engineered tissue is an assembloid containing ACM, AVCM and VCM organoids.

In a non-limiting embodiment, the assembloids or "mini hearts" simulate the conduction axis of the heart allowing evaluation of impulse propagation from the atrial to the ventricular end. Without intending to be limited by theory, assembloids generated from wildtype hiPSCs show remarkable features observed *in vivo* such as a "fast-slow-fast" activation pattern.

Without intending to be limited by theory, cardiac organoids can be prepared by culturing the AVC-like cardiomyocyte(s), ACM and/or VCM cardiomyocytes to create the organoids, followed by their fabrication into assembloids.

Methods of producing ACM and VCM organoids are known in the art. Cells typically compact within about 20 hours to about 30 hours, preferably about 24 hours forming contractile organoids. After another about 20 hours to about 30 hours, preferably about 24 hours, individual organoids may be combined to prepare assembloids in suitable culturing plates or flasks. Suitable plates include 24-well plates containing silicon inserts or flat bottom 96-well plates. Preferably, ACM, AVCM, or VCM organoids may then arranged such that they are in contact with one another, creating a cardiac assembloid. These cardiac assembloid perform synchronous contractions within 1 or 2 days. In some embodiments, cardiac assembloids may be created by arranging AVCM with other types of cardiac organoids.

In a non-limiting embodiment, the assembloids may be prepared by methods such as but not limited to 3D-bioprinting, self-aggregation and other patterning methods.

### Intended applications of the AVC-like cardiomyocyte(s) of the present disclosure

A further aspect of the present disclosure includes various uses of the AVC cardiomyocyte like cell(s). Applications include but are not limited to transplantation, for example for *in vivo* pacemaking as a biological pacemaker, disease modelling and testing candidate drugs. Other uses include studying the safety pharmacology testing of drugs for potential side effects on AVC-like cardiomyocytes and the development of AVC pacemaker unit from PSCs, MESP1/2 mesoderm cells or cardiac progenitor cells, and studying the physiology of human AVC cardiomyocyte pacemaker as healthy human samples are not readily available. The assembloids described above display appropriate gene expression and conduction patterns and are therefore, a valuable tool to study the AV conduction axis *in vitro.*

The present disclosure, accordingly, further provides a method of identifying a candidate drug comprising:
a. generating a population of the AVC-like cell(s) of the present disclosure as described in the above embodiments;
b. contacting the AVC-like cell(s) with a drug candidate;
c. measuring parameter(s) selected from a group comprising beat rate, action potential characteristics, and/or ion currents of the AVC-like cell(s) contacted with the drug candidate;
d. comparing the said parameter(s) to control AVC-like cell(s) not treated with the drug candidate; and
e. selecting the drug candidate which modulates the said parameter(s) compared to the control cell as the candidate drug.

In a non-limiting embodiment, the candidate drug is a drug for treating a disease selected from a group comprising Sinus Node Dysfunction or Sick Sinus Syndrome (SSS), bradycardia, tachycardia, sinus node arrest/block caused by aging (mostly fibrosis of the sinus node), electrical remodeling, or a genetic disorder.

**In** some embodiments, further envisaged herein is a method for identifying effects of mutations or pathogenic genetic variants on the functioning of cardiomyocytes or their response to therapy, said method comprising:
a. generating a population of the AVC-like cell(s) carrying the mutations or genetic variation of interest;
   b1. analyzing the functionality of the said AVC-like cell(s), or
   b2. contacting the said AVC-like cell(s) with a drug candidate, and measuring parameter(s) selected from a group comprising beat rate, action potential characteristics, and/or ion currents of the AVC-like cell(s) contacted with the drug candidate; and,
c. comparing the said functionality or the parameter(s) to wild-type or control AVC-like cell(s) not treated with the drug candidate, respectively.

Accordingly, in some embodiments, the present disclosure provides the isolated AVC-like cardiomyocyte(s) as obtained by any of the method(s) described in the above embodiments, for use in disease modelling.

Further envisaged herein is the isolated AVC-like cardiomyocyte(s) as obtained by any of the method(s) described in the above embodiments, for use as a medicament.

Further envisaged herein is the isolated AVC-like cardiomyocyte(s) as obtained by any of the method(s) described in the above embodiments, for use as a medicament for treating Atrioventricular block and disorders concerning myocytes of the atrioventricular conduction system.

The present disclosure further provides use of isolated AVC-like cardiomyocyte(s) as obtained by any of the method(s) described in the above embodiments in a drug discovery method.

The subject matter of embodiments of the present disclosure is described here with specificity to meet statutory requirements, but this description is not necessarily intended to limit the scope of the disclosure. The disclosed subject matter may be embodied in other ways, may include different elements or steps, and may be used in conjunction with other existing or future technologies. This description should not be interpreted as implying any particular order or arrangement among or between various steps or elements except when the order of individual steps or arrangement of elements is explicitly described.

### EXAMPLES

### Materials and Methods

### hiPSC lines

hiPSC lines, LUMC0099iCTRL#04 (Female) and LUMC0047iCTRL#1A (Male) used in this study were generated by the iPSC core facility of the Leiden University Medical Center.

hiPSC line from a 48-year-old female patient carrying LMNAc.1477C>T variant was generated after obtaining informed consent and approval of the medical ethics committee of the Amsterdam University Medical Center. Skin biopsy material was cultured on fibronectin (5 µg/ml; Corning, #356008) coated flask in BIO-AMF2 medium (Biological Industries, #01-194-1). Isolated primary fibroblasts were reprogrammed to hiPSCs using the CytoTune 2.0 Sendai Reprogramming Kit (ThermoFisher Scientific, #A16517) according to the manufacturer's instructions. LMNAc.1477C>T, clone D44 (Figures 12, 13 and 16; Figure 14) and LMNAc.1477C>T, clone A18 (Figure 15), were used in this study. A corresponding isogenic control line was generated by correcting the LMNAc.1477C>T mutation in clone D44 using the PE3 prime editing approach 46. In short, LMNAc.1477C>T hiPSCs were nucleofected with three plasmids, i.e. plasmid encoding the pCMV-PE2-P2A-GFP prime editor (Addgene, #132777), a plasmid encoding the pegRNA (Addgene, #132777) containing the template to correct the LMNAc.1477C>T variant and silence the PAM sequence, and a plasmid encoding the sgRNA (Addgene, #65777) for additional second nick. For nucleofection the Human Stem Cell Nucleofector Kit (Lonza, #VPH-5012) was used. Nucleofected iPSCs were plated as single cells on a 10 cm dish coated with growth factor reduced Matrigel (Corning, #356231). Colonies were manually picked for further expansion. Colonies were genotyped by Sanger Sequencing and stable karyotype confirmed by G-banding.

hiPSCs from all lines were maintained on growth factor reduced Matrigel-coated plates (Corning, #354230) in mTESR1 medium (Stem Cell Technologies, #85850) at 37°C / 5% CO2 and passaged once a week using 0.5 mM EDTA (Invitrogen, #15575-038).

### Differentiation of hiPSC lines to cardiomyocytes

For cardiomyocyte differentiation, hiPSCs were dissociated using 1x TryPLE Select (ThermoFisher, #12563011) and seeded at a density of 1.8-3.2 x104cells/cm2 in mTESR1 medium (Stem Cell Technologies, #85850). Differentiation was induced when cells reached 80 - 90% confluency. Mesoderm induction medium was composed of BPEL supplemented with 20 ng/mL Activin-A (Miltenyi Biotec, #130-115-012), 20 ng/mL BMP4 (R&D systems, #314-BP-101/CF) and 1.5 µM CHIR99021 (Axon Medchem, #1386). After 72 hours (day 3 of differentiation), medium was replaced with BPEL containing 5 µM XAV939 (TOCRIS, #3748/10). For VCM differentiation, XAV939 was left for 96 hrs. For SANCM, ACM, and AVCM differentiation, cells were treated with XAV939 for 24 hours followed by addition of subtype-specific factors (day 4 of differentiation). For SANCM, this included BPEL medium supplemented with 5 µM XAV939, 2.5 ng/mL BMP4, 5 µM SB431542 (TOCRIS, #1614), 250 nM RA (Sigma, #R2625-50MG), 250 nM PD173074 (Selleck Chemicals, #1264) and incubated for 48 hrs. ACM cells were treated with BPEL containing 1 µM BMS753 (Tocris, #3505) and 5 µM XAV939 for 72 hrs. For W+R (AVCM condition), cells were incubated with **BPEL** containing 5 ng/mL WNT2 (Sigma Aldrich, #SRP6560-10UG) and 250 nM RA (Sigma, #R2625-**50MG**) for 72 hrs. Media containing subtype specification factors were removed on day 6 or day 7 of differentiation (See Figure 1A). Henceforth, all cultures were refreshed every 3 - 4 days with BPEL media. Cells were used for downstream experiments from day 16 onwards.

### Flow Cytometry

Differentiated cardiomyocytes were dissociated using 1x TrypLE Select (ThermoFisher, #12563011). Cells were fixed using fixation buffer (Biolegend #420801) for 20 minutes at room temperature. Staining was performed with Cardiac Troponin T antibody (Miltenyi Biotec, #130-120-403, 1:50) or isotype control (Miltenyi Biotec #130-118-446, 1:50) diluted in 1x intracellular staining buffer (Biolegend #421002) and incubated on ice in the dark for 30 minutes. Acquisition was performed using FACS Symphony A1 (BD Biosciences). Data was analyzed using FLowJo v10.

### RNA isolation and quantitative Real-Time PCR (qRT-PCR)

Total RNA was isolated using Nucleospin RNA kit (Machery Nagel, # MN740955.50). cDNA was synthesized using Superscript II (ThermoFisher Scientific, #18064071) with oligo dT primers (125 µmol/L, Sigma) and dNTP set (100nM, Solis Biodyne, #02-21-00100) according to manufacturer's protocol. Each qPCR reaction contained 1x LightCycler 480 SYBR Green I Master (Roche, #4887352001), 1 µM primers (Forward and Reverse - Table 1) and cDNA (equivalent to 10 ng RNA). RT-qPCR Primer pairs were designed to span an exon-exon junction and/or at least one intron. Target sequences were amplified using the LightCycler 2.0 Real-Time PCR system (Roche Life Science) with the following amplification protocol: 5 min at 95 °C followed by 45 cycles of 10 sec at 95 °C, 20 sec at 60 °C, and 20 sec at 72 °C. Data analysis was performed using LinRegPCR program. Relative expression values were obtained by normalizing N0 values to the Geomean of two experimentally assessed reference genes, RPLP0 and GUSB.

**Table 1. RT-qPCR Primer pairs**

| **Target** | **Sequence (Forward and reverse primers)** |
|---|---|
| *ACTN2* | AACCACTTTGACAGGAGGAAGAA |
| | CTTGCCCGTTGGGATCTACC |
| *NKX2.5* | CCAAGGACCCTAGAGCCGAA |
| | ACACGTCTCACTCAGCATTTGT |
| *TBX18* | TTGCTAAAGGCTTCCGAGAC |
| | AGGTGGAGGAACTTGCATTG |
| *SHOX2* | CCATAAAGGTGTTCTCATAGGGGC |
| | AACCTGAAAGGACAAGGGCG |
| *NPPA* | CGATCTGCCCTCCTAAAAAGC |
| | TTGTCCTCCCTGGCTGTTATC |
| *NR2F2* | CCGAGTACAGCTGCCTCAA |
| | TTTTCCTGCAAGCTTTCCAC |
| *MYL2* | TACGTTCGGGAAATGCTGAC |
| | TTCTCCGTGGGTGATGATG |
| *MYH7* | AACACCAACCTGTCCAAGTTCC |
| | TGAGCAGATCAAGATGTGGCAA |
| *BMP2* | TATCGCAGGCACTCAGGTCA |
| | AACTCCTCCGTGGGGATAGAA |
| *MSX2* | GCCTCGGTCAAGTCGGAAAAT |
| | GGAGCTGGGATGTGGTAAAGG |
| *TBX2* | ACCCTGAGATGCCCAAACG |
| | AGTTTAGGATGGTGAAGCCGTG |
| *TBX3* | ACACTGGAAATGGCCGAAGA |
| | TGGGACATAAATCTTTGAGGTTCG |
| *HCN1* | ACGGTGTTGCTGGTGTCAT |
| | GTCCTTTGGTCAGCAGGCAA |
| *HCN4* | GATCCTCAGCCTCTTACGCC |
| | AGGAGCATCATGCCGATGAG |
| *KCNJ3* | TGTCGTCATCCTAGAAGGCA |
| | AAAAACGATGACCCCAAAGA |
| *CACNA1D* | CTCGCCCGTTTGCTATGATTC |
| | GGAAGATGGGAGACGACGG |
| *SCN5A* | CAGGCGAGTGTATTGTCAAGC |
| | GATGATGTCCGAGAGCACAGT |

### Cell sorting for scRNA-seq

Single cell sequencing was performed using SORT-seq approach. Cells from two independent differentiations were collected on day 19. Single cells were sorted into 384-well plates, each well containing an oil droplet with barcoded primers, spike-ins and dNTPs. Preparation of single-cell libraries was performed using the CEL-Seq2 protocol. Paired-end sequencing was performed on the NextSeq500 platform using 1x75 bp read length kit.

### Bioinformatic Analysis

### Reference genome annotation

Mapping was performed using BWA-MEM against the (human) genome assembly GRCh38 (hg38). Count matrices were generated using MapAndGo, filtering reads with a minimum quality score of 60 and no alternative hits.

### scRNA-seq data preprocessing, normalization and batch-correction, clustering, differential gene expression, cell-type identification, and visualization

Data analysis was performed using the R toolkit Seurat version 4. Quality control and preprocessing, dimensional reduction, clustering, and differential gene expression were performed according to the standard workflow (https://satijalab.org/seurat/). Briefly, high quality single cells collected on D19 were selected according to the following parameters: gene count > 1,000 and < 8,000 and mitochondrial gene count < 50%. Normalization, scaling, and identification of variable features (nfeatures=3000) based on variance stabilizing transformation ("vst") was performed using the SCTransform command. Since technical plate-to-plate variations were observed, SCTransform data integration was performed by normalizing each dataset individually, identifying integration anchors within the datasets collected on the same timepoint and integrating the datasets. The same approach was used for integrating our in vitro dataset with in vivo datasets after converting human genes to mouse orthologues using the R package "babelgene". Mouse datasets published in de Soysa et al., 2019 and Hill et al., 2019 were retrieved and cardiomyocyte clusters were extracted using TNNT2 and ACTN2 expression. Mouse cardiomyocyte populations and hiPSC-AVCM (genes converted to mouse orthologues) were merged and analyzed together. Dimensionality reduction was performed using principal component (PC) analysis and Uniform Manifold Approximation and Projection (UMAP) with the top 25 PCs (hiPSC-derived cardiomyocyte subtype datasets, Figure 1 and Figure 4), top 20 PCs (clustering of W+R-hiPSC-CM, Figure 1 and integration of in vivo and in vitro datasets, Figure 5 and Figure 6) and seed set to 2020. For cell clustering, a KNN (K-nearest neighbor) graph was constructed based on euclidean distance in PCA space and clusters were identified using the Louvain algorithm, as implemented in the FindNeighbors and FindClusters command. Identified clusters were then visualized in a UMAP using the DimPlot command. For differential expression testing and visualization, LogNormalization was performed according to the standard workflow on the uncorrected dataset and differential gene expression was determined using Wilcoxon rank sum test. Differentially expressed gene lists show genes, which are expressed in at least 25% in either of the two fractions of cells and limited to genes, which are differentially expressed (on average) by at least 0.25-fold (log-scale) between the two compared cell fractions. Cell type specific marker genes were used to annotate cell clusters. VlnPlot, FeaturePlot and DoHeatMap commands were used to visualize gene expression. For gene module score calculation, the Top 50 differentially expressed genes of several datasets (Schmidt et al., 2024, Farah et al., 2024, Ye et al., 2024 and Kanemaru et al., 2024) were used as input features for the AddModuleScore command.

### Immunocytochemistry of 2D cardiomyocyte monolayers

Differentiated cardiomyocytes were dissociated using 1x TrypLE Select (ThermoFisher Scientific #12563011) and seeded at a density of 4.0 x 105 per coverslips (12 mm, Epredia, #CB00120RA020MNZ0). Cells were fixed four days later with 4% paraformaldehyde (VWR, #28794.295). Cells were permeabilized with 0.1% Triton-X (Sigma, T8787). A blocking step was performed with 4% normal swine serum (Jackson Immunoresearch, #014-000-121) for one hour at room temperature. Primary and secondary antibodies (Tables 2 and 3) were diluted in 4% normal swine serum and incubated at room temperature for 1 hour. Nuclei were stained with DAPI (Sigma Aldrich #D9542). Image acquisition was performed using the LEICA confocal SP8-X DLS lightsheet microscope. Image visualization and processing was performed with LAS-X (Leica) software. Details of antibodies used are provided in Star Methods.

**Table 2. Primary antibodies**

| **Antibody** | **Manufacturer** | **Catalog number** | **Application** | **Dilution** |
|---|---|---|---|---|
| Alpha Actinin (*ACTN2*) | Sigma | A7811 | Monolayer | 1:800 |
| *TBX2* | Peprotech | 169030-1-AP | Monolayer | 1:150 |
| *MSX2* | Millipore | HPA005652-25UL | Monolayer | 1:200 |

**Table 3. Secondary antibodies**

| **Antibody** | **Manufacture r** | **Catalog number** | **Application** | **Dilution** |
|---|---|---|---|---|
| Donkey anti-Rabbit Alexa 647 | Invitrogen | A31573 | Monolayer Assembloids | 1:250 |
| Donkey anti-Mouse Alexa 488 | Invitrogen | A21202 | Monolayer Assembloids | 1:250 |

### Single Cell Patch-Clamp

Differentiated cardiomyocytes at day 16 were dissociated using 1x TryPLE Select (ThermoFisher Scientific #12563011) and seeded at a density of 7.0x103 per coverslip (Epredia, #CB00120RA020MNZ0). Single cell patch-camp was performed seven days later. Cells with a smooth surface and intact membrane, which displayed spontaneous activity were chosen for measurements. Action potentials were recorded at 37°C with the amphotericin-B-perforated patch-clamp technique using a Axopatch 200B Clamp amplifier (Molecular Devices Corporation). Measurements were carried out in Tyrode's solution containing 140 mM NaCl, 5.4 mM KCl, 1.8 mM CaCl2, 1.0 mM MgCl2, 5.5 mM glucose and 5.0 mM HEPES. pH was adjusted to 7.4 with NaOH. Pipettes (borosilicate glass; resistance 1.5-2.5 MΩ) were filled with a solution containing 125 mM K-gluconate, 20 mM KCl, 10 mM NaCl, 0.4 mM amphotericin-B and 10 mM HEPES, pH was adjusted to 7.2 with KOH. Signals were low-pass-filtered (cutoff frequency 10 kHz) and digitized at 40 kHz. Potentials were corrected for the estimated change in liquid junction potential. Data acquisition and analysis were performed using custom software, Scope4PC. DAD susceptibility was tested with procedures as described 33,34 and DADs were defined as 1 mV or larger depolarization deflections.

I_{Na} was activated by 50 ms depolarizing pulses from -120 mV using modified solutions at 37°C. Extracellular solution contained 130 mM NaCl, 10 mM CsCl, 1.8 mM CaCl2, 1.2 mM MgCl2, 11.0 mM glucose, 5.0 mM HEPES. pH was adjusted to 7.4 with CsOH. 5 µM nifedipine was added to block ICa,L. Pipettes were filed with 133 mM CsCl, 3.0 mM NaCl, 2.0 mM MgCl2, 2.0 mM Na2ATP, 2.0 mM TEACl, 10 mM EGTA, 5.0 mM HEPES. pH was adjusted to 7.2 with CsOH. If was measured as ivabradine-sensitive current (3 µM) using the same solutions as used for the action potential measurements and 2 sec hyper- and depolarizing voltage clamp steps from a holding potential of -40 mV. Currents were normalized to cell capacitance.

### Preparation of cardiac spheroids and their fabrication into assembloids

ACM, AVCM, or VCM cardiomyocytes on day 16 were dissociated using 1x TryPLE Select and plated at 10 × 104/well in low adherence, V-bottom 96-well plates (Griener, #651161). Cells typically compacted within 24 hours forming contractile spheroids. After another 24 hours, individual spheroids were combined to prepare assembloids in 24-well plates containing silicon inserts (ibidi #80209) or flat bottom 96-well plates (Griener, #655185). Spheroids were serially pipetted in close proximity and arranged such that they were in contact with one another. Only one assembloid was cultured per well and media changes were performed every other day. Synchronous contractions were observed within 1 - 2 days.

### Optical Mapping

Spheroids or Assembloids, four-five days after preparation, were incubated in media containing 20 µM Di-4 ANEPPS (Anaspec Inc, #AS-84723) and 1 µM Blebbistatin (Cayman Chemical, #13013) at 37°C for 25 minutes. For mapping, spheroids/assembloids were transferred to a water bath containing modified Tyrode's solution prepared as described above with pH maintained at 7.4 by equilibration with a mixture of 95% O2 and 5% CO2. Excitation light was provided by a 5 W power LED (filtered 510 ± 20 nm). Fluorescence (filtered>610 nm) was transmitted through a tandem lens system on CMOS sensor (100x100 elements, sampling rate 5 kHz, MICAM Ultima). Optical action potentials were visualized using Brainvision Inc software and analyzed using Rhythm and Orca software. Pacing of assembloids was provided by a twin tipped micro electrode (900 µA, 2 ms pulse) and custom-made OT & Sien Heart Stimulator software.

### Immunocytochemistry of spheroids and assembloids

Single spheroids or assembloids were fixed seven days after fabrication with 4% paraformaldehyde (VWR, #28794.295) were imbedded in 1% agarose (Sphaero Q, #S103B) followed by paraffin imbedding. The paraffin-imbedded single spheroids or assembloids were sectioned at 5 µm. Sections were mounted onto silane-coated slides, deparaffinized in xylene, rehydrated in graded ethanol series and washed in phosphate-buffered saline (PBS, pH 7.4). Heat-induced antigen retrieval was performed using unmasking solution (Vector Labs #H-3300-250). A blocking step was performed with 4% bovine serum albumin (BSA; Sigma-Aldrich #A7906) for one hour at room temperature. Afterwards, sections were incubated with primary antibodies diluted in 4% bovine serum albumin (BSA; Sigma-Aldrich #A7906) overnight at 4°C. After washing in phosphate-buffered saline (PBS, pH 7.4), sections were incubated with fluorochrome-conjugated secondary antibodies and DAPI (Sigma-Aldrich #D9542) in the dark at room temperature for 2 hr. Image acquisition was performed using the LEICA Thunder Wide Field Fluorescence Microscope. Image visualization and processing was performed with LAS-X (Leica) software. Details of antibodies used are provided below.

### [Ca²⁺]^{I} Measurements

Day 16 cardiomyocytes were dissociated, and measurements performed 5 - 7 days later. Cells were loaded with 1 µM Indo-1 AM (Cayman Biotech) for 20 minutes at 37°C in Tyrode's Solution. Cells were subsequently washed with the same Tyrode's solution to remove excess Indo-1. Dual wavelength emission (405 nM/505 nM) of Indo-1 AM after excitation at 340 nm was recorded at 37°C in Tyrode's solution. [Ca2+]I transients were elicited at 0.7 Hz using field stimulation. Signals were digitized at 1 kHz, filtered at 100 Hz, and correct for background fluorescence.

[Ca²⁺]ⁱ and SERCA, NCX, and slow extrusion exchange measurements and calculations were performed. Data acquisition and analysis were accomplished using custom software.

### Note:

### Statistical Analysis

Statistical analysis was carried out in GraphPad (www.graphpad.com) Prism version 9.1.0 for Windows. Data are represented as mean ± standard error of the mean (s.e.m.). Non-parametric tests were performed in all cases. Number of samples (n) and the method used to test statistical significance are stated in each figure legend. contains additional details of group comparisons and P values obtained.

### Access to scRNA-seq data and scripts used for analysis

scRNA-seq data of hiPSC-derived AVCM is deposited in NCBI GEO repository under the accession number GSE250173 and the token "upwpeeioxlyhrcd". scRNA-seq data of hiPSC-derived SANCM, ACM and VCM is available in GSE189782. R scripts are available on Github and can be accessed via https://gitfront.io/r/awiesinger/Mkgw6DNhFuBW/cardiac-assembloid/.

### Example 1: WNT2 and RA steer mesoderm to atrioventricular cardiomyocyte fate

To direct differentiation toward AVCM, the general principles of steering subtype specification in differentiating hiPSCs were followed, which relies on modulating specific signaling pathways at the cardiac mesoderm stage (Figure 1A). Based on knowledge from embryonic heart development in animal models, it was hypothesized that activation of WNT and retinoic acid (RA) signaling pathways would drive AVCM fate. To this end, cardiac mesodermal cells were treated at day 4 with WNT ligand WNT2 and RA (W+R). For comparison, other cardiomyocyte subtypes were generated, i.e. sinoatrial nodal (SANCM), atrial (ACM) and ventricular cardiomyocytes (VCM) from hiPSCs using previously described protocols. Contractile cardiomyocytes were typically observed in all groups around day 10. Flow cytometry analysis of terminally differentiated cardiomyocytes (day 18 - 20 of differentiation), revealed the presence of 70 - 90% TNNT2⁺ cardiomyocytes in all four groups (Figure 1B). mRNA expression of classic cardiomyocyte lineage marker genes and of genes associated with specific subtypes was assessed (Figure 2). Pan muscle marker ACTN2 was expressed in all subtypes, albeit lower in SANCM and W+R cardiomyocytes. NKX2-5 was similarly expressed in ACM, VCM, W+R groups and was expectedly lower in SANCM, in which only a subset of cells express this transcription factor. Importantly, the expression of BMP2, MSX2, TBX2 and TBX3, genes implicated in AVCM development were predominantly expressed in W+R cardiomyocytes. Immunostaining also confirmed that the expression of TBX2 and MSX2 at the protein level was also predominant in W+R cardiomyocytes compared with other subtypes (Figure 3A, 3B). Furthermore, analysis of TBX18 and SHOX2 (SANCM lineage), NPPA and NR2F2 (atrial lineage) and MYL2 and MYH7 (ventricular lineage) suggest that the W+R cultures contain both cells of AVCM identity, as well as potentially a fraction of cells with chamber identity (Figure 2).

To determine the transcriptome and composition of W+R cultures, single cell RNA sequencing (scRNA-seq) of differentiated cells was performed at day 19 (Figure 1C - 1H). For the analysis, transcriptome data from W+R cells were combined with our previously published datasets constituting hiPSC-derived SANCM, ACM and VCM resulting in a total of 2412 cells. Unsupervised clustering identified 12 clusters excluding 2 clusters (cluster 8 and 13) with dead cells/ambient RNA (Figure 1C). Based on the expression of TNNT2 and ACTN2 (Figure 1D), it was determined that clusters 0 - 4, 6 and 11 represented cardiomyocytes. However, cardiomyocytes in cluster 11 appeared to be at a different cell cycle stage, as identified by the expression of several cyclin dependent kinase genes and was excluded from further analysis. Clusters 5, 7, 9, 10 and 12 were comprised of non-cardiomyocytes (*COL1A1*⁺*, COL1A2*⁺*, COL6A3*⁺*, VIM*⁺ mesenchymal cells/fibroblasts in Cluster 5; *TBX18*⁺*, IGFBPS⁺, RDH10⁺, KRT18⁺* proepicardial cells in Cluster 7; three small clusters (9, 10 and 12) of unknown identity.

The six cardiomyocyte clusters were extracted (Figure 4A, B) and annotated according to their gene expression patterns. Cardiomyocytes resulting from W+R treatment predominantly contributed to a distinct cluster (Cluster 2) (Figure 4C), characterized by the expression of AVCM associated genes TBX2, TBX3, MSX2 as well as RSPO3 (Figure 4D). Cluster 2 also differentially expressed GATA6, NKX2-5 and TBX5, transcription factors crucial for heart development, with particular significance to AVCM development and function. The gene expression signature of Cluster 2 was unique compared with the other cardiomyocyte subtypes such as SANCM (Cluster 1; SHOX2+/ISL1+), SV (Cluster 4; SHOX2⁺/TCF21⁺/COL3A1⁺), ACM (Cluster 0; NPPA⁺/NR2F2⁺), VCM (Cluster 3; MYL2⁺/HOPX⁺) and outflow tract, OFT (Cluster 6; ISL1⁺/HAPLN1⁺/PITX2⁺) (Figure 4E-I). To determine the composition of cells obtained from W+R treatment in more detail, secondary clustering of this group was performed including all of the cells obtained from this differentiation protocol, which identified four clusters (Figure 1E). The majority of cells obtained from W+R treatment are comprised of cardiomyocytes expressing *TNNT2* and *ACTN2,* distributed in Clusters 0 (45%) and cluster 1 (24%) respectively (Figure 1E, 1F). Furthermore, both these clusters expressed AVCM-associated genes *TBX2, TBX3* and *EMP2* (Figure 1G). However, a key difference was noticed between the two clusters. In addition to AVCM associated genes, Cluster 1 differentially expressed atrial genes *NPPA, HEY1, MYL7* and *MYL4* (Figure 1G, H), which is a feature of cells in the atrial transition zone of the AV conduction axis. Lastly, Cluster 2 (20% of W+R population) and 3 (11% of W+R population) were identified as non-cardiomyocytes as they lacked expression of cardiomyocyte genes (Figure 1F, 1H). Whilst Cluster 2 could be identified as proepicardial-like cell population based on the expression of *WT1, TBX18* and *BNC1* (Figure 1G, 1H), it was not possible to determine the identity of Cluster 3, which was the smallest cluster. In summary, transcriptional characterization of W+R cells suggests they are distinct from other hiPSC-derived cardiomyocyte subtypes and their gene expression is suggestive of AVCM-like identity.

### Example 2: Cells generated from WNT2 and RA treated cultures recapitulate molecular signature of in vivo AVCM.

To test the correlation between W+R cardiomyocytes to *in vivo* cell types, publicaly available scRNA-seq datasets were utilized. As hiPSC-derived cardiomyocytes are expected to best correlate with fetal hearts, W+R population was first compared to datasets from mouse hearts of embryos ranging from embryonic day 9.25 to 13.5. For the integration of human and mouse data, human gene names were converted to their mouse orthologues using the R package "babelgene". After integration and clustering, five cardiomyocyte clusters were identified in the murine heart datasets (Figure 5A). The identity of these clusters was ascertained as AVCM (Figure 5B; Figure 6A); atrial (ACM); combined sinus venosus and sinoatrial nodal (SV_SANCM) population which share developmental origin and phenotypic properties; ventricular (VCM), and outflow tract (OFT) cardiomyocytes (Figure 6A, 6B), respectively, based on marker gene expression. This approach revealed that around 65% of W+R cardiomyocytes clustered together with murine AVCM (Figure 5C, 5D). About 13% and 20% of the cells clustered with murine ACM or VCM, respectively (Figure 5D). To further confirm this finding, top 50 differentially expressed genes of mouse AVC (compared to mouse ACM, SV_SANCM, OFT and VCM) were extracted and utilized to assess gene module scoring. Among the different hiPSC-derived cardiomyocyte subtypes (UMAP from Figure 4A), highest correlation of the Top 50 mouse AVCM genes was observed with the W+R population, reiterating their AVC-like identity (Figure 5E). Similarly, the correlation of transcript signatures of W+R cardiomyocytes to recently reported human fetal and adult AV node was also assessed. The Top 50 differentially expressed genes extracted from these human datasets and subsequent gene module scoring confirmed that W+R cardiomyocytes correlate closely with the human fetal and adult AV node (Figure 5F).

Next, the comparability between AVCM generated in this study and two other recently published studies that presented the derivation of AVCM from hiPSCs or human embryonic stem cells (hESCs) respectively was assessed. Gene module scoring of their datasets was first performed using Top 50 genes from the mouse, human fetal and human adult AV canal/node to ascertain identity (Figure 6C, D). The AVCM population from Schmidt et al associated with the Top 50 genes from the in vivo datasets (Figure 6C). However, the cell type reported in Ye et al did not show meaningful correlation (Figure 6D). To gain further insight into the comparability of AVCM from Schmidt et al and AVCM from this study, gene module scoring was performed using their Top 50 genes (Figure 6E). This analysis shows that their top genes best correlated with a subset of the AVCM of the present disclosure (Figure 6E; 6F). Overall, comparison of W+R treated cardiomyocytes to in vivo AVCM confirms their lineage identity. W+R cardiomyocytes are referred to as AVCM hereafter.

### Example 3: Electrophysiological analysis reveals hiPSC-AVCM recapitulate salient properties of in vivo AV nodal cardiomyocytes.

Differences in ion channel expression contribute to distinct electrophysiological properties of individual cardiomyocyte subtypes. Furthermore, cardiomyocytes of the sinoatrial and AV nodes share features that facilitate pacemaking properties. hiPSC-derived AVCM expressed *HCN1* and *HCN4,* responsible for I_{f}, which is implicated in diastolic depolarization of nodal cells, albeit lower than SANCM (Figure 7A). Similarly, *KCNJ3,* which contributes to I_{KACh}, as well as *CACNA1D* involved in I_{Ca,L}, in nodal as well as atrial cells were expressed in AVCM (Figure 7A).

Next, the action potential properties (Figure 7B) of AVCM was evaluated by single cell patch-clamp and compared them to SANCM. Representative action potential traces are shown in Figure 7C. To gain insights into the extent to which they recapitulate the salient differences seen in *in vivo* nodal cells, patch-clamp data of E17.5 murine nodal cells was included, considering the challenges in obtaining cells from the human heart. Representative action potential traces of murine nodal cells are shown in Figure 7D. Whilst cycle lengths in hiPSC-derived nodal cells were comparable, murine SANCM had shorter cycle lengths compared with murine AVCM (Figure 7E, 7F). Both hiPSC-AVCM and murine AV nodal cells exhibited a more negative maximal diastolic potential (MDP) (-68.3 ± 1.1 mV in hiPSC-AVCM; -66.1 ± 1.8 mV in murine-AVCM) compared with their respective SANCM counterparts (-63.3 ± 1.9 mV in hiPSC-SANCM; -59.2 ± 2.0 mV in murine-SANCM). Upstroke velocities in murine nodal cells were comparable. Small but significantly faster upstroke velocities were noted in hiPSC- AVCM compared with hiPSC-SANCM. In addition, it was observed that observed that action potential amplitude (APA) was significantly higher in both human and murine AV groups (86.9 ± 3.2 mV in hiPSC-AVCM; 94.9 ± 3.9 mV in murine-AVCM) compared with SANCM (73.0 ± 2.4 mV in hiPSC-SANCM; 76.8 ± 6.4 mV in murine-SANCM). Lastly, action potential duration (APD) at 20% (APD₂₀), 50% (APD₅₀) and 90% (APD₉₀) repolarization was comparable between the nodal cell types in both mouse and hiPSC groups (Figure 7E,7F). The results demonstrated that hiPSC-derived AVCM recapitulate salient electrophysiological properties of *in vivo* AVCM.

In addition, the action potential properties of hiPSC-derived AVCM were compared to hiPSC-derived ACM and VCM (Figure 8). Representative traces are shown in Figure 8A. Cycle lengths in AVCM were shorter compared with VCM, but not significantly different from ACM (Figure 8B). Average MDP in ACM was more negative than AVCM and VCM, albeit not significant. Upstroke velocities in AVCM were slower than VCM and comparable to ACM. In addition, APA in AVCM and ACM were similar and were significantly lower than in VCM. Lastly, APD₂₀ and APD₅₀ in AVCM were significantly different from both ACM and VCM, whilst APD₉₀ only differed from VCM (Figure 8B). In conclusion, differences in action potential properties between AVCM and chamber subtypes, particularly VCM were apparent and confirms their distinctive identity.

### Example 4: AVCM generated from an independent wildtype line demonstrates reproducibility.

To determine the robustness of the WNT2 and RA based approach for driving AVCM differentiation, an independent wildtype hiPSC line was utilized to generate ACM, AVCM and VCM subtypes. Expression of *ACTN2* was similar in all three groups (Figure 9A). In addition, preferential expression of *MSX2* and *TBX2* along with lower expression of chamber genes *NPPA* and *MYL2* corroborated the AVCM identity of the resulting cells (Figure 9A), further supported by their ion channel gene profile (Figure 9B). Expression of *HCN1, HCN4, KCNJ3,* and *CACNA1D* was higher in AVCM compared with the chamber subtypes. In addition, expression of *SCN5A,* which predominantly contributes to I_{Na}, was lower in AVCM.

Electrophysiological properties measured using single cell patch-clamp (Figure 9C) revealed the nodal nature of these cells as evidenced by the prominent diastolic depolarization (Phase 4) as well as slower upstroke velocities (Figure 9D, 9E) and were largely in agreement with the data shown in Figure 7 and Figure 8. In an effort to further characterize some of the ion currents contributing to action potential characteristics of AVCM, voltage clamp experiments were performed. I_{Na}, which distinguishes chamber cells from nodal cells was significantly lower in AVCM compared with ACM and VCM (Figure 9F), in agreement with the lower mRNA expression of *SCN5A.*

Furthermore, voltage clamp measurements of I_{f} (Figure 9G) as well as the response to 3 µM ivabradine, which prolonged cycle length in AVCM (Figure 9H) demonstrated that I_{f} contributes to automaticity in AVCM. In addition to I_{f}, I_{KAch} has been shown to be important for modulation of pacemaking activity. 10 µM carbachol, which activates I_{KAch}, prolonged the cycle length in AVCM and ACM, where this current is expected to be present (Figure 9I). In summary, molecular, and functional characterization demonstrated the translatability of WNT2 and RA driven AVCM differentiation to an independent hiPSC line.

### Example 5: Cardiac assembloids simulate conduction patterns observed in vivo.

Next, the behavior of AVCM was assesed in 3-dimensional (3D) tissues, particularly in a setting where their role in the conduction of electrical activity from the atria to the ventricles could be studied. To this end, ACM, AVCM and VCM spheroids were prepared from the respective subtype cultures as schematically represented in Figure 10A. 100K, 250K and 500K cells were tested for spheroid aggregation. Spheroids made of higher cell number formed empty cores and did not aggregate properly. Therefore, 100K was determined to be the most optimal cell number for making spheroids. Cells dissociated at day 16 of differentiation and resuspended in low adherence plates formed spheroids typically within 24 - 48 hours (Figure 10B).

Optical mapping of individual subtype spheroids revealed slower propagation in AVCM (1.84 ± 0.25 cm/s) compared with ACM (5.27 ± 0.70 cm/s) and VCM (8.30 ± 0.68 cm/s) (Figure 11A). Immunostaining further confirmed subtype specific expression patterns in the spheroids (Figure 11B-D). Expression of NR2F2 was predominant in the ACM spheroid (Figure 11B). Expression of MYL2 was only observed in the VCM spheroid (Figure 11C). Importantly, MSX2 was preferentially expressed in the AVCM spheroid (Figure 11D).

To create a three-component tissue model, the assembloid approach was implemented as previously described for neuronal organoids. 48 hours after fabrication, individual spheroids were allowed to fuse together in microwell plates to enable the formation of so-called "cardiac assembloids" composed of ACM, AVCM and VCM in physiological order (Figure 10C). Impulse propagation in these tissue constructs was assessed five days later using optical mapping. When assembled in the morphological order of the vertebrate heart tube, i.e. AVCM sandwiched between ACM and VCM (Figure 10C), impulse initiation was observed from the ACM end in at least 60% of the assembloids (Figure 10D). The propagation was unidirectional and followed a "fast-slow-fast" activation pattern (ACM 4.78 ± 0.61 cm/s, AVCM 1.41 ± 0.22 cm/s, VCM 5.42 ± 0.73 cm/s), reminiscent of heart tube in vertebrate embryos (Figure 10D) ³¹. To further confirm that slower conduction is an intrinsic property of AVCM independent of their position within the assembloids, constructs were fabricated with ACM and AVCM towards the periphery and VCM spheroids in the center (Figure 10E). In these tissues, impulses were once again initiated primarily from the ACM end. The propagation in these assembloids followed a "fast-fast-slow" pattern (ACM 3.70 ± 0.60 cm/s, VCM 3.74 ± 0.51 cm/s, AVCM 1.45 ± 0.19 cm/s) (Figure 10F). To further strengthen then observations, "Quad assembloids" were also generated containing all four cardiomyocyte subtypes in the physiological order, i.e. SANCM, followed by ACM, AVCM, and VCM. In 90% of the constructs, pacing began consistently in the SANCM end (Figure 11E). The propagation followed a "slow-fast-slow-fast" pattern, as expected from nodal and chamber-like tissues. Lastly, immunofluorescence analysis of subtype regions in the assembloids (Figure 10G, H) confirmed respective identities, demonstrating congruence with observations from single spheroids prior to fabrication. Overall, these findings demonstrate that assembloids display appropriate gene expression and conduction patterns and are therefore, a valuable tool to study the AV conduction axis *in vitro.*

### Example 6: LMNAc.1477C>T assembloids display conduction block.

The ability of assembloids to recapitulate important features of the AV conduction axis prompted us to examine whether they would model AV block caused by pathogenic variants in the *LMNA* gene. A heterozygous carrier of *LMNAc.1477C>T* (LMNAp.Q493X) presented with atrial tachycardia, paroxysmal atrial fibrillation, and 1^{st} degree AV block (Figure 12A), combined with episodes of 2^{nd} degree AV block (Mobitz I). Informed consent of the patient and institutional ethical approvals were obtained to generate a hiPSC line that carried the C>T substitution at position 1477. A corresponding isogenic control line was created by correcting the point mutation using prime editing (Figure 12B).

Assembloids were generated from both *LMNAc.1477C>T* and isogenic hiPSCs. In both the groups, impulses were initiated from the ACM end in at least 60% of the constructs (Figure 12C), comparable to data from wildtype line shown in Figure 10. However, upon pacing at 2 Hz, impulses ended abruptly at the interface between AVCM and VCM spheroids in 40% of the *LMNAc.1477C>T* assembloids, which increased to over 80% when paced at 3 Hz (Figure 12D). Representative propagation maps at 3 Hz, demonstrating impulse block are shown in in Figure 12E. To ascertain whether the observed impulse block phenotype is due to impaired AVCM function, assembloids were created where *LMNAc.1477C>T* AVCM spheroid was sandwiched by ACM and VCM from the isogenic line. Spontaneous impulse generation was observed from the ACM spheroid which progressed towards the VCM end of the assembloid, also in this setting (Figure 12F). When paced at 2 Hz and 3 Hz, the propagation of electrical impulses ceased at the interface between AVCM and VCM spheroids in 50% and 75% of the constructs, respectively (Figure 12G). Representative propagation maps at 3 Hz, demonstrating impulse block are shown in in Figure 12H. These findings suggest that intrinsic defects in AVCM underlie the conduction block phenotype in LMNA assembloids.

### Example 7: Alterations in intracellular calcium homeostasis characterize LMNAc.1477C>T AVCM

To examine the cause of impulse block in AVCM harboring a pathogenic variant in LMNA, the expression of ion channel and calcium handling genes were assessed. Compared with isogenic AVCM, a decrease in the expression of intracellular calcium handling genes *RYR2, SERCA2A, NCX1,* and *CASQ2* (Figure 13A) was observed. Expression of membrane channel genes *CACNA1D, HCN4,* or *KCNJ3* were not significantly different between the two groups (Figure 14A).

Following up on these findings, a single cell patch-clamp as well as assays to determine intracellular calcium ([Ca²⁺]ᵢ) homeostasis were performed. Representative action potential traces of *LMNAc.1477C>T* and isogenic AVCM are shown in Figure 13B. Analysis of action potential properties revealed prolonged cycle length and longer APD₉₀ in *LMNAc.1477C>T* AVCM compared with the isogenic control. All other parameters were comparable (Figure 13C). Next, the susceptibility of *LMNAc.1477C>T* to triggered activity was tested. A fast-pacing protocol was used followed by an 8 second pause and dynamic clamp methodology. The number of delayed afterdepolarizations (DADs) was significantly higher in cardiomyocytes generated from *LMNA*c.1477C>T hiPSCs (Figure 14B). Noticeably, the propensity for DADs was significantly higher in AVCM compared with the other subtypes (Figure 14C) suggesting an intracellular calcium overload in this cell type. Key electrophysiological findings including impulse block in assembloids, as well as alterations in action potential properties and increased propensity for DADs were also observed in AVCM derived from an additional *LMNAc.1477C>T* clone (Figure 15).

To determine whether alterations in intracellular calcium homeostasis are indeed present in AVCM, [Ca²⁺]ᵢ transient measurements of *LMNA* and isogenic AVCM was performed. Figure 13D shows typical [Ca²⁺]ᵢ transient recordings of *LMNA* and isogenic AVCMs following 0.7 Hz electrical stimulation. A summary of average [Ca²⁺]ᵢ characteristics is presented in Figure 13E. Diastolic [Ca²⁺]ᵢ was increased in *LMNA* AVCM as compared with the isogenic control while there were no significant differences in systolic [Ca²⁺]ᵢ or amplitude of the transients. Mean rise time of calcium [Ca²⁺]ᵢ was not significantly different between *LMNA* AVCM and isogenic control. Notably, the decay of the [Ca²⁺]ᵢ transient was significantly slower in *LMNA* AVCM as indicated by significant increase in time constant (tau) of decay (Figure 13E).

To better understand the cause of the slower decay time in *LMNA* AVCM, measured the activity of sarcoplasmic reticulum (SR), Ca²⁺ ATPase (SERCA), Na⁺-Ca²⁺ exchanger (NCX) was measured, and the slow mechanisms (mitochondrial Ca²⁺ uniporter and sarcolemmal Ca²⁺ ATPase) in ([Ca²⁺]ᵢ) sequestering by the application of caffeine and Nickel chloride (NiCl₂) pulses ³⁴. Representative transients of isogenic and *LMNA* AVCM are shown in Figure 13F. The amplitude of caffeine-evoked transients in the presence of NiCl₂ was reduced in *LMNA* AVCM (Figure 13G), indicating a reduction in SR calcium. Neither fractional SR Ca²⁺ release nor the relative contributions of SERCA, NCX and slow mechanisms differed significantly between groups (Figure 13G). However, the rate constant of SERCA2A (*K*_{SERCA}) and the rate constant of NCX (*K*_{NCX}) denoting their activity, were significantly slower in *LMNA* AVCM, indicating that both mechanisms contribute to the slower calcium transient decay. Taken together, *LMNA*c.1477C>T AVCM exhibit impaired calcium handling, which can contribute to reduced excitability and compromise conduction relay.

### Example 8: Propagation block in LMNAc.1477C>T AVCM is rescued by application of Rycal Stabilizer, S107

Reduced Casq2 is associated with increased leakiness of Ryr channels ³⁵. Furthermore, the detection of elevated diastolic calcium, evidenced by DADs as well as calcium transient measurements in LMNA1477C>T AVCM prompted us to test whether S107 ³⁶, which stabilizes the binding affinity of RYR channels with Calstabin-1 (FKBP12) and diminish leakiness would rescue the phenotype. Application of 10 µM S107 to LMNA1477C>T assembloids paced at 3 Hz resolved propagation block in 4 out of 5 assembloids. In addition, treatment with S107 also resulted in a reduction in the amount of DADs by more than two fold. In conclusion, these results demonstrate the power of hiPSC-derived AVCM and assembloids for revealing molecular, cellular, and functional causes underlying complex conduction disorders.

Additional embodiments and features of the present disclosure will be apparent to one of ordinary skill in art based on the description provided herein. The embodiments herein provide various features and advantageous details thereof in the description. Descriptions of well-known/conventional methods and techniques are omitted so as to not unnecessarily obscure the embodiments herein.

The foregoing description fully reveals the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein, without departing from the principles of the disclosure.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

### References

∘ Wiesinger, A., Li, J., Fokkert, L., Bakker, P., Verkerk, A. O., Christoffels, V. M., Boink, G. J. J., & Devalla, H. D. (2022). A single cell transcriptional roadmap of human pacemaker cell differentiation. eLife, 11, e76781. https://doi.org/10.7554/eLife.76781*.*
∘ Li, J., Wiesinger, A., Fokkert, L., Boukens, B. J., Verkerk, A. O., Christoffels, V. M., Boink, G. J. J., & Devalla, H. D. (2022). Molecular and electrophysiological evaluation of human cardiomyocyte subtypes to facilitate generation of composite cardiac models. Journal of Tissue Engineering, 13, 20417314221127908. https://doi.org/10.1177/20417314221127908*.*
∘ de Soysa, T. Y., Ranade, S. S., Okawa, S., Ravichandran, S., Huang, Y., Salunga, H. T., Schricker, A., Del Sol, A., Gifford, C. A., & Srivastava, D. (2019). Single-cell analysis of cardiogenesis reveals basis for organ-level developmental defects. Nature, 572(7767), 120-124. https.//doi.org/10.1038/s41586-019-1414-x*.*
∘ Hill, M. C., Kadow, Z. A., Li, L., Tran, T. T., Wythe, J. D., & Martin, J. F. (2019). A cellular atlas of Pitx2-dependent cardiac development. Development (Cambridge, England), 146(12), dev180398. https.//doi.org/10.1242/dev.180398*.*
∘ Farah, E. N., Hu, R. K., Kern, C., Zhang, Q., Lu, T. Y., Ma, Q., Tran, S., Zhang, B., Carlin, D., Monell, A., Blair, A. P., Wang, Z., Eschbach, J., Li, B., Destici, E., Ren, B., Evans, S. M., Chen, S., Zhu, Q., & Chi, N. C. (2024). Spatially organized cellular communities form the developing human heart. Nature, 627(8005), 854-864. https://doi.org/10.1038/s41586-024-07171-z
∘ Kanemaru, K., Cranley, J., Muraro, D., Miranda, A. M. A., Ho, S. Y., Wilbrey-Clark, A., Patrick Pett, J., Polanski, K., Richardson, L., Litvinukova, M., Kumasaka, N., Qin, Y., Jablonska, Z., Semprich, C. I., Mach, L., Dabrowska, M., Richoz, N., Bolt, L., Mamanova, L., Kapuge, R., ... Teichmann, S. A. (2023). Spatially resolved multiomics of human cardiac niches. Nature, 619(7971), 801-810. hitps://doi.org/10.1038/s41586-023-06311-1
∘ Schmidt, C., Deyett, A., Ilmer, T., Haendeler, S., Torres Caballero, A., Novatchkova, M., Netzer, M. A., Ceci Ginistrelli, L., Mancheno Juncosa, E., Bhattacharya, T., Mujadzic, A., Pimpale, L., Jahnel, S. M., Cirigliano, M., Reumann, D., Tavernini, K., Papai, N., Hering, S., Hofbauer, P., & Mendjan, S. (2023). Multi-chamber cardioids unravel human heart development and cardiac defects. Cell, 186(25), 5587-5605.e27. https.//doi.org/10.1016/j.cell.2023.10.030

## Claims

1. A method of generating AVC-like cell(s) from cardiogenic mesoderm cells, said method comprising:
incubating cardiogenic mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

2. The method according to claim 1, wherein the cardiogenic mesoderm cells are MESP1/2 mesoderm cells expressing MESP1 and/or MESP2 and optionally, EOMES.

3. The method according to claim 2, wherein the MESP1/2 mesoderm cells are prepared by incubating pluripotent stem cells (PSCs) in an early mesoderm induction medium comprising an activator of WNT signalling and optionally, a BMP ligand and/or a TGFb superfamily protein such as Activin A to generate the MESP1/2 mesoderm cells.

4. The method according to any one of claims 1-3, comprising:
incubating pluripotent stem cells (PSCs) in an early mesoderm induction medium comprising an activator of WNT signalling and optionally, a BMP ligand and/or a TGFb superfamily protein to generate the MESP1/2 mesoderm cells; and
incubating the MESP1/2 mesoderm cells in an AVC cardiomyocyte induction medium comprising WNT2 and retinoic acid (RA) to obtain the AVC-like cell(s), wherein the AVC-like cell(s) express *TBX3* and at least one of *BMP2, MSX2* and *TBX2.*

5. The method according to any one of claims 1-4, wherein the AVC cardiomyocyte induction medium comprises about 1 ng/mL to about 20 ng/mL WNT2 and about 50 nM to about 1000 nM RA.

6. The method according to any of claims 1-5, wherein the AVC cardiomyocyte induction medium optionally further comprises a BMP ligand, wherein the BMP ligand includes *BMP4* and/or *BMP2.*

7. The method according to any one of claims 1 to 6, wherein the cardiogenic mesoderm cells are incubated in the AVC cardiomyocyte induction medium comprising WNT2 and retinoic acid (RA) for about 24 hours to about 96 hours.

8. AVC-like cell(s) obtainable by the method according to any of claims 1-7.

9. AVC-like cell(s) expressing at least *TBX3* and one of *BMP2, MSX2, TBX2.*

10. The AVC-like cell(s) according to claim 8 or 9, wherein the AVC-like cell(s) exhibit one or more electrophysiological properties of AVC cardiomyocytes, wherein the said one or more electrophysiological properties are selected from exhibition of HCN dependent funny current, I_{f} or response to ivabradine, which targets I_{f} and response to carbachol, which activates inward rectifier potassium current, I_{KACH}.

11. An engineered tissue comprising the AVC-like cell(s) according to claim 8 or 9.

12. The engineered tissue according to claim 11, further comprising Atrial cardiomyocytes (ACM) and Ventricular cardiomyocytes (VCM).

13. The engineered tissue according to any of claims 11 or 12, wherein said engineered tissue is an organoid containing AVC-like cell(s) according to any of claims 7-9 (AVCM), Atrial cardiomyocytes (ACM) and/or Ventricular cardiomyocytes (VCM).

14. The engineered tissue according to claim 13, wherein said engineered tissue is an assembloid containing the AVCM, the ACM and/or the VCM organoids.

15. A method of identifying a candidate drug comprising:
a. generating AVC-like cell(s) according to the method of claims 1-7;
b. contacting the AVC-like cell(s) with a drug candidate;
c. measuring parameter(s) selected from a group comprising beat rate, action potential characteristics, and/or ion currents of the AVC-like cell(s) contacted with the drug candidate;
d. comparing the said parameter(s) to a control AVC-like cell(s) not treated with the drug candidate; and
e. selecting the drug candidate which modulates the said parameter(s) compared to the control cell as the candidate drug.

16. The AVC-like cell(s) as obtained by the method according to any one of claims 1-7, for use as a medicament or in disease modelling.

17. A method for identifying effects of mutations or pathogenic genetic variants on the functioning of cardiomyocytes or their response to therapy, said method comprising:
a. generating a population of AVC-like cell(s) according to the method of claims 1-7,
wherein the said cells carry the mutations or genetic variation of interest;
b1. analyzing the functionality of the said AVC-like cell(s), or
b2. contacting the said AVC-like cell(s) with a drug candidate, and measuring parameter(s) selected from a group comprising beat rate, action potential characteristics, and/or ion currents of the AVC-like cell(s) contacted with the drug candidate; and,
c. comparing the said functionality or the parameter(s) to wild-type or control AVC-like cell(s) not treated with the drug candidate, respectively.

18. Use of the AVC-like cell(s) as obtained by the method according to any one of claims 1-7, in a drug discovery method.
